# EUROPEAN PATENT SPECIFICATION

(11) **EP 4 005 480 B1**
(45) Date of publication and mention of the grant of the patent: **23.08.2023**
(21) Application number: 21206614.6
(22) Date of filing: 05.11.2021
(51) Int. Cl.: A61B 5/24, A61B 5/25, A61B 5/291

(54) **AN ELECTRODE ARRANGEMENT, A NEURAL PROBE, AND A METHOD FOR MANUFACTURING AN ELECTRODE ARRANGEMENT**
ELEKTRODENANORDNUNG, NEURALE SONDE UND VERFAHREN ZUR HERSTELLUNG EINER ELEKTRODENANORDNUNG
AGENCEMENT D'ÉLECTRODES, SONDE NEURONALE, ET PROCÉDÉ DE FABRICATION D'UN AGENCEMENT D'ÉLECTRODES

(30) Priority: 16.11.2020 EP 20207697
(43) Date of publication of application: 01.06.2022
(73) Proprietor: IMEC vzw, 3001 Leuven (BE)
(72) Inventor: Hiblot, Gaspard, 3000 Leuven (BE); Van der Plas, Geert, 3000 Leuven (BE)
(74) Representative: AWA Sweden AB

(56) References cited:
- US-A1- 2013 167 360
- US-A1- 2015 208 969
- US-A1- 2016 360 991
- US-A1- 2020 196 950

## Description

### Technical field

The present inventive concept relates to an electrode arrangement for providing high quality signal recording. In particular, the present inventive concept relates to electrode arrangements for use in neural signal recording.

### Background

Arrays of electrodes may be used in various applications, e.g. for enabling acquisition of signals with a spatial resolution. This may for instance be used for studying propagation of electrical signals.

The array of electrodes may be arranged on a surface for acquiring signals. However, it may in some applications be useful to have two arrays of electrodes arranged on opposite surfaces of a substrate. Thus, the substrate may record signals on two sides. This may for instance be useful in analyzing activity and/or characteristics of biological matter, wherein the substrate may be arranged to extend into the biological matter and in contact with the biological matter on the two opposite surfaces of the substrate. By having arrays of electrodes on the opposite surfaces, extra signals may be recorded such that additional information may be acquired.

It may be beneficial to provide at least some processing of the recorded electrical signals close to the electrodes. For instance, the electrical signals may need to be preamplified before the signals are transferred to more advanced processing circuitry in order to allow high quality signal recording. Thus, a substrate carrying an array of electrodes may need electronic circuitry in close relation to the array of electrodes. Having two arrays of electrodes on opposite surfaces of the substrate, it may thus be necessary to solve how to connect the arrays of electrodes formed on opposite surfaces of the substrate to electronic circuitry.

In manufacturing of electrode arrangements, there is a risk of damaging electronic circuitry by a large floating electrode being etched by plasma etching while the electrode is connected to the electronic circuitry, so called plasma induced damage (PID). Thus, PID issues may also be taken into account when connecting the arrays of electrodes formed on opposite surfaces of the substrate to electronic circuitry,

Electrode arrangements for providing signal recording are known from US2013/167360, US2015/208969, US2020196950 or US2016/360991.

### Summary

It is an object of the present inventive concept to provide an electrode arrangement that enables recording electrical signals by arrays of electrodes on opposite sides of a substrate. In particular, it is an object of the present inventive concept to enable processing of electrical signals recorded on opposite sides of the substrate in an electronic circuitry arranged on the substrate close to the electrodes. In some embodiments, it is an object of the present inventive concept to allow manufacturing of electrode arrangements having arrays of electrodes on opposite sides of a substrate while avoiding plasma induced damage (PID).

These and other objects of the invention are at least partly met by the invention as defined in the independent claims. Preferred embodiments are set out in the dependent claims.

According to a first aspect, there is provided an electrode arrangement, comprising: a semiconductor carrier substrate having a first side surface and a second side surface opposite to the first side surface; a first array of electrodes arranged above the first side surface; a second array of electrodes arranged below the second side surface; an electronic circuitry for processing electrical signals recorded by the first array of the electrodes and the second array of electrodes, said electronic circuitry comprising at least one layer arranged above the first side surface and below the first array of electrodes; a connecting layer arranged above the at least one layer of the electronic circuitry, said connecting layer being configured to connect the electrodes of the first array and the electrodes of the second array to the electronic circuitry, wherein the connecting layer is configured to provide a first connection in a plane of the connecting layer between a first point and a second point for connecting an electrode in the second array to the electronic circuitry; a first interconnect for electrically connecting the first point to the electronic circuitry; a plurality of through-substrate vias, TSVs, extending through the semiconductor carrier substrate between the first side surface and the second side surface for forming an electrical connection through the semiconductor carrier substrate; a second interconnect extending through a plane defined by the at least one layer of the electronic circuitry, wherein the second interconnect and a first TSV of the plurality of TSVs electrically connect the second point to the electrode in the second array.

Thanks to the electrode arrangement, a semiconductor carrier substrate is provided with a first and a second array of electrodes on opposite surfaces of the substrate, wherein the first and second arrays of electrodes are connected to electronic circuitry on the substrate. This ensures that electrical signals may be recorded on opposite sides of the substrate while allowing processing of the electrical signals close to the electrodes.

The electrode arrangement comprises electronic circuitry arranged above the first side surface of the semiconductor carrier substrate. The second array of electrodes arranged below the second side surface of the semiconductor carrier substrate is connected to the electronic circuitry on the first side surface. This ensures that the electronic circuitry may be manufactured while processing only one side of the substrate, which may ensure that the manufacture of the electronic circuitry may be relatively simple.

The electrical connection of an electrode of the second array to the electronic circuitry is achieved by a TSV providing a connection through the substrate, a second interconnect providing a connection between the TSV and the connecting layer, the connecting layer providing a connection between a second point connected to the second interconnect and a first point, and a first interconnect providing a connection between the first point in the connecting layer and the electronic circuitry. The electrode of the second array may be directly connected to the TSV by being directly arranged below the second side surface of the substrate. However, the electrode of the second array may alternatively be connected to the TSV through an additional interconnect extending between the second side surface of the substrate and the electrode.

The connecting layer may be formed as a back-end-of-line (BEOL) layer, whereas the electronic circuitry may be formed in front-end-of-line (FEOL) layers. Thanks to the connection of the electrode of the second array to the connecting layer, the electrodes arranged on a backside of the substrate may be connected to electronic circuitry on a frontside of the substrate through a BEOL layer on the frontside.

As used herein, a definition that a layer or structure is arranged "above" or "below" another layer or structure should be interpreted in relation to a plane as defined by the substrate when the substrate is oriented with plane of the substrate extending horizontally such that a plane defined by the electronic circuitry is arranged above the plane of the substrate. It should be realized that the term "above" or "below" does not necessarily mean directly above or directly below. Rather, intermediate layers and/or structures may be provided in-between a layer or structure is arranged "above" or "below" another layer or structure.

The semiconductor carrier substrate may be a silicon carrier substrate, such as a silicon wafer. However, it should be realized that other semiconductor materials may be used instead.

The connecting layer may comprise an electrically conducting material for providing an electrical connection between the first point and the second point in the connecting layer. The electrically conducting material may be a metal. The connecting layer may further comprise a dielectric material, such as an oxide, between structures of conducting material for electrically isolating separate structures of conducting material in the connecting layer.

The first and second interconnects may each provide an electrical connection between two points and the interconnects may be formed by an electrically conducting material embedded in dielectric material.

The first and second interconnects may extend between layers such that when the plane of the substrate is oriented to extend horizontally, the first and second interconnects may extend vertically in the electrode arrangement. The first and second interconnects may e.g. be vias in the electrode arrangement.

The electrode arrangement may comprise a plurality of connections for connecting a plurality of electrodes in the second array of electrodes to the electronic circuitry. Thus, for each electrode in the second array of electrodes that is connected to the electronic circuitry, there may be a separate first connection, a separate first interconnect, a separate TSV and a separate second interconnect for electrically connecting the electrode to the electronic circuitry. However, it should be realized that each electrode in the second array of electrodes need not necessarily be connected to the electronic circuitry, such that some electrodes of the second array may for instance be connected by a wire parallel to a plane of the substrate to circuitry for processing signals which may be arranged in a separate area of the substrate or on another physical substrate.

The electronic circuitry may be manufactured as FEOL layers on the semiconductor substrate and the connecting layer may be manufactured as a BEOL layer on the semiconductor substrate. This may be particularly useful for processing the semiconductor substrate and for manufacturing of the electrode arrangement. However, it should be realized that the electronic circuitry and the connecting layer need not necessarily be formed as FEOL layers and BEOL layer, respectively.

According to an embodiment, the connecting layer is configured to provide a second connection in a plane of the connecting layer between a third point and a fourth point for connecting an electrode in the first array to the electronic circuitry, the electrode arrangement further comprising a third interconnect for electrically connecting the third point to the electronic circuitry, a fourth interconnect for connecting the fourth point to a second TSV of the plurality of TSVs, a fifth interconnect for connecting the electrode in the first array to a third TSV of the plurality of TSVs, and a bridging structure arranged at the second side surface for connecting the second TSV to the third TSV.

The electrical connection of an electrode of the first array to the electronic circuitry is achieved by a fifth interconnect connecting the electrode to a third TSV providing a connection through the substrate, a bridging structure connecting the third TSV to a second TSV providing a connection back to a first side surface of the substrate, a fourth interconnect providing a connection between the second TSV and the connecting layer, the connecting layer providing a connection between a fourth point connected to the fourth interconnect and a third point, and a third interconnect providing a connection between the third point in the connecting layer and the electronic circuitry. Thus, the electrode of the first array may be connected to the electronic circuitry through a connecting structure extending through the substrate.

The electrodes of the first array may be formed before the bridging structure is connected to third TSV and the second TSV such that the electrodes of the first array are not connected to the electronic circuitry when being formed. This implies that the plasma etching of the electrodes of the first array will not cause PID of the electronic circuitry since the electrodes are not electrically connected to the electronic circuitry during plasma etching. Hence, PID issues may be avoided.

The bridging structure may be formed on a bottom carrier substrate, which is separate from the semiconductor carrier substrate of the electrode arrangement. This bottom carrier substrate may be bonded to the semiconductor carrier substrate after the first array of electrodes have been formed such that the electrodes are not connected to the electronic circuitry during plasma etching. The bridging structure may comprise an electrically conducting material for providing an electrical connection between the second TSV and the third TSV. The electrically conducting material may be a metal.

The third, fourth and fifth interconnects may each provide an electrical connection between two points and the interconnects may be formed by an electrically conducting material embedded in dielectric material.

The third, fourth and fifth interconnects may extend between layers such that when the plane of the substrate is oriented to extend horizontally, the first and second interconnects may extend vertically in the electrode arrangement. The third, fourth and fifth interconnects may e.g. be vias in the electrode arrangement.

The connection between the third and fourth points used in connecting an electrode of the first array to the electronic circuitry and the connection between the first and second points used in connecting an electrode of the second array to the electronic circuitry may be provided in the same connecting layer. This may be beneficial in manufacturing of the electrode arrangement as the electrodes of the first and second arrays may be connected to a common layer. However, it should be realized that different connecting layers may be used for providing connections between the third and fourth points and for providing connections between the first and second points. For instance, different BEOL layers may be used.

The bridging structure may be provided as a lateral connection in a single layer arranged below the second side surface of the semiconductor carrier substrate. However, it should be realized that the bridging structure may be provided by an electrical connection extending through a plurality of layers.

According to an embodiment, the connecting layer is configured to provide a second connection in a plane of the connecting layer between a third point and a fourth point for connecting an electrode in the first array to the electronic circuitry, the electrode arrangement further comprising a third interconnect for electrically connecting the third point to the electronic circuitry, and a fourth interconnect for connecting the electrode in the first array to the fourth point.

In this embodiment, the electrical connection of an electrode of the first array to the electronic circuitry is achieved by a fourth interconnect connecting the electrode to the connecting layer, the connecting layer providing a connection between a fourth point connected to the fourth interconnect and a third point, and a third interconnect providing a connection between the third point in the connecting layer and the electronic circuitry. Thus, the electrode of the first array may be connected to the electronic circuitry through a connecting structure extending between a plane defined by the electronic circuitry and a plane in which the first array of electrodes is arranged, i.e. the connecting structure does not extend through the substrate.

The third and fourth interconnects may each provide an electrical connection between two points and the interconnects may be formed by an electrically conducting material embedded in dielectric material.

The third and fourth interconnects may extend between layers such that when the plane of the substrate is oriented to extend horizontally, the third and fourth interconnects may extend vertically in the electrode arrangement. The third and fourth interconnects may e.g. be vias in the electrode arrangement.

The electrodes of the first array may be formed on a separate top carrier substrate such that the electrodes of the first array are not connected to the electronic circuitry when being formed. Rather, the top carrier substrate may be bonded to the semiconductor substrate after the electrodes have been formed. This implies that the plasma etching of the electrodes of the first array will not cause PID of the electronic circuitry since the electrodes are not electrically connected to the electronic circuitry during plasma etching. Hence, PID issues may be avoided.

According to an embodiment, an electrode in the first array of electrodes has a smaller area at a side facing the electronic circuitry than at a side facing away from the electronic circuitry. This may be a result of the electrode being processed on a separate top carrier substrate, wherein the etch is not perfectly anisotropic such that sloping sidewalls of the electrodes may be formed.

According to an embodiment, a barrier structure is arranged above the first array of electrodes. A conformal barrier structure or seed structure may be deposited before metal deposition for forming the array of electrodes in order to increase adherence of the metal on supporting material (i.e. provide a seed function) and to prevent metal from escaping into surrounding oxide (i.e. provide a barrier function). Thus, a barrier structure being arranged above the first array of electrodes may be a result of the electrode being processed on a separate top carrier substrate.

However, even if the electrodes in the first array of electrodes are connected to the electronic circuitry through a connecting structure extending between a plane defined by the electronic circuitry and a plane in which the first array of electrodes are arranged, i.e. the connecting structure does not extend through the substrate, the electrodes need not necessarily be formed on a separate top carrier substrate in order to avoid PID issues. According to an alternative, PID issues may be resolved by adding electrostatic discharge protection to the electrodes. However, this may degrade accuracy of measurements using the electrodes.

According to an embodiment, the electrode arrangement further comprises a back-end-of-line capacitance structure formed at the second side surface between the second array of electrodes and the semiconductor carrier substrate.

This implies that back-end-of-line capacitances may be provided at the second side surface so as to gain layout space at the first side surface of the substrate.

According to a second aspect, there is provided a neural probe, said neural probe comprising the electrode arrangement according to the first aspect, wherein the semiconductor carrier substrate is adapted for being inserted into a brain.

Effects and features of this second aspect are largely analogous to those described above in connection with the first aspect. Embodiments mentioned in relation to the first aspect are largely compatible with the second aspect.

The electrode arrangement according to the first aspect may be particularly useful in a neural probe. Thanks to having electrodes on opposite sides of a substrate, recording of signals may be made on both sides of the substrate such that more information may be acquired in order to provide improved analysis of activity and/or characteristics of the brain. Also, in order to minimize effects of insertion of the neural probe into the brain, the neural probe should be as small as possible. By having electrodes on both sides of the substrate, the neural probe may be small while enabling acquisition of a large amount of information.

The semiconductor carrier substrate may for instance comprise a pointed tip, which may facilitate insertion of the semiconductor carrier substrate into the brain.

The neural probe may be intended for insertion into a human brain for acquiring of information for analyzing activity and/or characteristics of the human brain. However, the neural probe may also or alternatively be intended for insertion into an animal brain, such as a brain of a mammal.

According to a third aspect, there is provided a method for manufacturing an electrode arrangement, said method comprising: forming an electronic circuitry above a first side surface of a semiconductor carrier substrate, said electronic circuitry comprising at least one layer; forming a plurality of metal-filled structures extending from the first side surface into the semiconductor carrier substrate; forming a connecting layer above the electronic circuitry and forming interconnects between the connecting layer and the electronic circuitry and between the connecting layer and the metal-filled structures such that the connecting layer is configured to provide a first connection in a plane of the connecting layer between a first point connected by a first interconnect to the electronic circuitry and a second point connected by a second interconnect, extending through a plane defined by the at least one layer of the electronic circuitry, to a first metal-filled structure of the plurality of metal-filled structures; forming a first array of electrodes above the electronic circuitry; thinning the semiconductor carrier substrate to define a second side surface of the semiconductor carrier substrate opposite to the first side surface and to expose a bottom of the metal-filled structures so that the metal-filled structures form a plurality of through-substrate vias, TSVs, extending through the semiconductor carrier substrate; forming a second array of electrodes at the second side surface of the semiconductor carrier substrate, wherein an electrode of the second array of electrodes is electrically connected by a first TSV of the plurality of TSVs and the second interconnect to the second point in the connecting layer.

Effects and features of this third aspect are largely analogous to those described above in connection with the first and second aspects. Embodiments mentioned in relation to the first and second aspects are largely compatible with the third aspect.

Thanks to the manufacturing method, a semiconductor carrier substrate is manufactured having a first and a second array of electrodes on opposite surfaces of the substrate, wherein the first and second arrays of electrodes are connected to electronic circuitry on the substrate. This ensures that electrical signals may be recorded on opposite sides of the substrate while allowing processing of the electrical signals close to the electrodes.

The electronic circuitry is formed above the first side surface of the semiconductor carrier substrate. The second array of electrodes arranged below the second side surface of the semiconductor carrier substrate is connected to the electronic circuitry on the first side surface. This ensures that the electronic circuitry may be manufactured while processing only one side of the substrate, which may ensure that the manufacture of the electronic circuitry may be relatively simple.

Thanks to the forming of a plurality of metal-filled structures extending from the first side surface into the semiconductor carrier substrate, the semiconductor carrier substrate is prepared for providing TSVs. When the semiconductor carrier substrate is thinned a bottom of the metal-filled structures is exposed such that the TSVs are provided.

When the TSVs are provided, the second array of electrodes may be formed at the second side surface of the substrate and the connection of the second array of electrodes to the TSVs will connect the electrodes in the second array to the electronic circuitry. The forming of the second array of electrodes at the second side surface of the semiconductor carrier substrate may be achieved by bonding a separate substrate to the semiconductor carrier substrate or by processing the semiconductor carrier substrate at the second side thereof.

As used herein, the term "forming" should be interpreted as any way of creating the structure, such as by semiconductor processing which may involve one or more steps of deposition and etching of material and/or by bonding of substrates on which structures have been created.

According to an embodiment, said forming of the second array of electrodes comprises: processing a bottom carrier substrate, wherein said processing comprises forming the second array of electrodes on the bottom carrier substrate and defining electrical connection structures at a surface on the bottom carrier substrate, each electrical connection structure being arranged at or being electrically connected to an electrode in the second array of electrodes; and bonding the surface of the bottom carrier substrate to a second side surface of the semiconductor carrier substrate by hybrid bonding.

Thanks to the second array of electrodes being formed by processing on a bottom carrier substrate, the electrodes in the second array may be formed while the electrodes are not connected to the electronic circuitry. This implies that the plasma etching of the electrodes of the second array will not cause PID of the electronic circuitry since the electrodes are not electrically connected to the electronic circuitry during plasma etching. Hence, PID issues may be avoided.

The second side surface of the semiconductor carrier substrate may expose a bottom of the metal-filled structures forming a plurality of through-substrate vias, TSVs. The second side surface of the semiconductor carrier substrate may further be provided with an oxide surface. This oxide surface may prevent shorting of metal structures (the second array of electrodes) on the bottom carrier substrate to the semiconductor substrate. Thus, the semiconductor carrier substrate may be covered by an oxide layer at the second side surface before bonding, wherein the metal-filled structures forming TSVs extend through the oxide layer so as to enable providing a connection to the electrodes of the second array. The hybrid bonding of the bottom carrier substrate to the second side surface may thus be performed by bonding the bottom carrier substrate to the oxide surface with metal-filled structures forming TSVs exposed at the oxide surface at the second side surface of the semiconductor carrier substrate.

The hybrid bonding may form bonds between metal structures and between oxide structures simultaneously, such that each of the surfaces being bonded may expose metal structures and oxide structures.

The metal structures at the surface of the bottom carrier substrate may thus be an electrical connection structure in the hybrid bonding. This electrical connection structure may constitute the electrodes of the second array of electrodes. However, according to an alternative, the electrical connection structure may be connected to the electrodes of the second array, such that the electrodes of the second array need not necessarily be formed at the surface of the bottom carrier substrate which is bonded to the semiconductor carrier substrate.

According to an embodiment, forming the connecting layer and forming interconnects comprises forming the connecting layer to provide a second connection in a plane of the connecting layer between a third point connected by a third interconnect to the electronic circuitry and a fourth point connected by a fourth interconnect to a second metal-filled structure of the plurality of metal-filled structures, wherein the method further comprises forming a fifth interconnect for connecting an electrode in the first array of electrodes to a third metal-filled structure of the plurality of metal-filled structures.

The fifth interconnect may thus provide an electrical connection of the electrode in the first array of electrodes to the third metal-filled structure. Also, an electrical connection between the electronic circuitry to the second metal-filled structure is provided through the third interconnect, the connecting layer and the fourth interconnect. The first array of electrodes may be formed by deposition and etching of electrode material while the third metal-filled structure is not connected to the second metal-filled structure. Thus, the electrodes of the first array are not connected to the electronic circuitry when being formed. This implies that the plasma etching of the electrodes of the first array will not cause PID of the electronic circuitry since the electrodes are not electrically connected to the electronic circuitry during plasma etching. Hence, PID issues may be avoided.

According to an embodiment, the processing on the bottom carrier substrate comprises defining an electrical bridging connection structure at a surface on the bottom carrier substrate, wherein said electrical bridging connection structure when the surface on the bottom carrier substrate is bonded to the second side surface of the semiconductor carrier substrate forms an electrical connection between a second TSV and a third TSV, which second and third TSVs are formed by the second and third metal-filled structures, respectively.

When the semiconductor carrier substrate is thinned, the second and third metal-filled structures may form a second and a third TSV, respectively. Thus, the bridging structure being provided on the bottom carrier substrate may connect the second and the third TSV when the bottom carrier substrate is bonded to the second side surface. This implies that a connection of the electrodes of the first array to the electronic circuitry may be closed when the bottom carrier substrate is bonded to the second side surface.

According to another embodiment, the forming of the first array of electrodes comprises: processing a top carrier substrate, wherein said processing comprises forming the first array of electrodes on the top carrier substrate, each electrode in the first array of electrodes being arranged at or being electrically connected to an electrical connection structure at a surface on the top carrier substrate; and bonding the surface on the top carrier substrate to a top surface above the first side surface of the semiconductor carrier substrate by hybrid bonding.

The electrodes of the first array may thus be formed on a separate top carrier substrate such that the electrodes of the first array are not connected to the electronic circuitry when being formed. Rather, the top carrier substrate may be bonded to the semiconductor substrate after the electrodes have been formed. This implies that the plasma etching of the electrodes of the first array will not cause PID of the electronic circuitry since the electrodes are not electrically connected to the electronic circuitry during plasma etching. Hence, PID issues may be avoided.

The metal structures at the surface of the top carrier substrate may thus be an electrical connection structure in the hybrid bonding. This electrical connection structure may constitute the electrodes of the first array of electrodes. However, according to an alternative, the electrical connection structure may be connected to the electrodes of the first array, such that the electrodes of the first array need not necessarily be formed at the surface of the top carrier substrate which is bonded to the semiconductor carrier substrate.

According to an embodiment, the method further comprises forming a planar oxide surface on the first array of electrodes on the semiconductor carrier substrate and bonding an oxide surface on a top carrier substrate to the planar oxide surface of the semiconductor carrier substrate by dielectric bonding.

According to this embodiment, the first array of electrodes may be formed by deposition and etching of electrode material on the semiconductor carrier substrate. In this case, PID issues may be avoided by the first array of electrodes being connected to the electronic circuitry through the second and third TSVs. Alternatively, PID issues may be resolved by adding electrostatic discharge protection to the first array of electrodes.

The bonding of the top carrier substrate to the planar oxide surface may facilitate that thinning of the semiconductor carrier substrate may be performed.

According to an embodiment, the method further comprising after said thinning of the semiconductor carrier substrate and said forming of the second array of electrodes, removing the top carrier substrate to expose a front surface of the electrode arrangement at which the first array of electrodes is arranged.

The top carrier substrate may be provided to ensure that the semiconductor carrier substrate may be thinned and that the second array of electrodes may be formed. It should be realized that manipulating substrates having a small thickness, such as below 100 µm thick, may be very difficult and that having the top carrier substrate bonded to the planar oxide surface ensures that the substrate to be handled during manufacturing is not too thin.

After thinning, the top carrier substrate may be removed such that the front surface at which the first array of electrodes is arranged may be exposed.

According to an embodiment, the method further comprising bonding the front surface to a temporary wafer.

When the front surface has been exposed, there may still be a need to remove a bottom carrier substrate. In order to allow removal of such bottom carrier substrate, e.g. by chemical-mechanical polishing, the front surface may need to be attached to a temporary wafer.

The front surface may be attached to the temporary wafer e.g. by an adhesive layer providing an attachment that may be easily removed while still providing sufficient strength to enable processing of the electrode arrangement attached to the temporary wafer.

According to an embodiment, the method further comprises, after said bonding of the front surface to the temporary wafer, removing the bottom carrier substrate to expose a back surface of the electrode arrangement at which the second array of electrodes is arranged.

Hence, the second array of electrodes may be exposed at the back surface of the electrode arrangement.

The method may then further include removing of the temporary wafer such that the first array of electrodes is exposed at the front surface and the second array of electrodes is exposed at the back surface.

According to an embodiment, forming of the first array of electrodes and forming of the second array of electrodes comprises plasma etching.

The method of manufacturing of the electrode arrangement may allow use of plasma etching for forming the first and the second arrays of electrodes, since risk of PID due to plasma etching may be handled by e.g. ensuring that the electrodes are not connected to the electronic circuitry when the plasma etching is performed.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 is a schematic view of a cross-section of an electrode arrangement according to a first embodiment.
Fig. 2 is a schematic view of a cross-section of an electrode arrangement according to a second embodiment.
Fig. 3 is a schematic view of a neural probe according to an embodiment.
Figs 4a-n are schematic views of cross-sections in manufacturing steps for manufacturing the electrode arrangement according to a method of a first embodiment.
Figs 5a-b are schematic views of cross-sections in manufacturing steps for manufacturing the electrode arrangement according to a method of a second embodiment.
Figs 6a-f are schematic views of cross-sections in manufacturing steps for manufacturing the electrode arrangement according to a method of a third embodiment.

### Detailed description

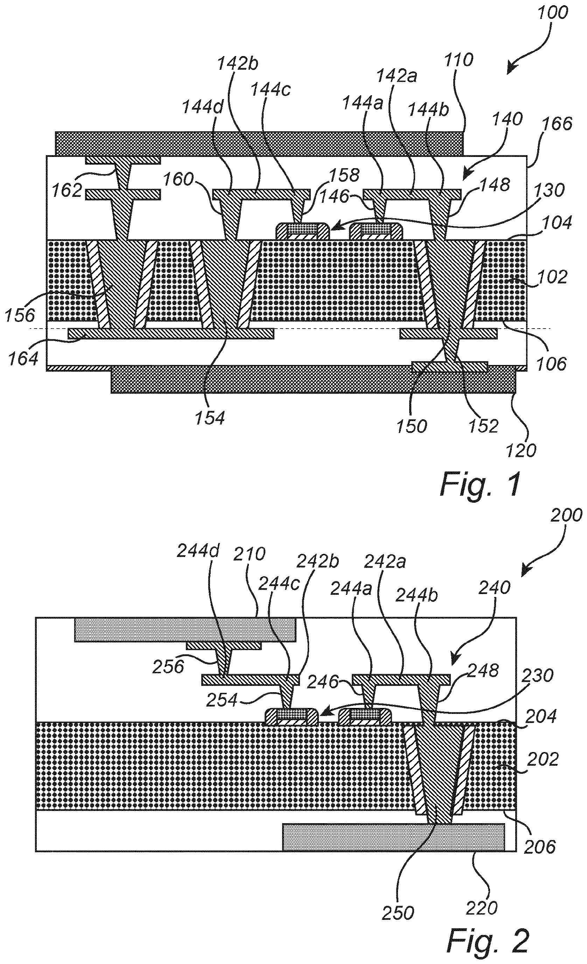

Fig. 1 illustrates an electrode arrangement 100 according to a first embodiment. The electrode arrangement 100 comprises a semiconductor carrier substrate 102, for instance a silicon substrate, having a first side surface 104 and a second side surface 106 opposite to the first side surface 104. In Fig. 1, a first electrode 110 is shown above the first side surface 104 and a second electrode 120 is shown below the second side surface 106, illustrating that the electrode arrangement 100 provides electrodes 110, 120 on a front side and a back side of the substrate 102.

It should be realized that the electrode arrangement 100 may comprise a first array of first electrodes 110 arranged above the first side surface 104 and a second array of second electrodes 120 arranged below the second side surface 106. However, for clarity, only one first electrode 110 and one second electrode 120 is illustrated in Fig. 1.

The first electrode 110 and the second electrode 120 may be formed by titanium nitride (TiN). This may be particularly useful for recording signals in biological matter, such as a brain. However, it should be realized that other materials may be used for the first electrode 110 and/or the second electrode 120.

The electrode arrangement 100 further comprises an electronic circuitry 130 for processing electrical signals recorded by the first electrode 110 and the second electrode 120.

The electronic circuitry 130 may be formed as an integrated circuit on the semiconductor substrate 102 for defining the processing of the electrical signals performed by the electronic circuitry 130. Thus, the electronic circuitry 130 may for instance comprise a plurality of transistors connected for providing desired processing of the electrical signals recorded by the electrodes 110, 120.

For instance, the electronic circuitry 130 may be configured to amplify the signals recorded by the electrodes 110, 120. A preamplifier close to the electrode 110, 120 may for instance be useful for enabling high quality neural signal recording. The pre-amplified signal may then be transferred to further processing units, such as other electronic circuitry for further processing of the signal.

In a neural probe, it may not be desired to have advanced signal processing circuitry arranged close to the electrodes 110, 120, since the size of the neural probe that is to be inserted into the brain needs to be as small as possible to minimize damage to brain tissue when the neural probe is inserted into the brain. Further, the signals may need to be transferred a relatively large distance from the electrodes 110, 120 to processing circuitry arranged outside the brain. In such case, it is beneficial to pre-amplify the recorded signal before it is transferred to processing circuitry outside the brain so as to ensure high quality neural signal recording.

The electronic circuitry 130 may be formed by front-end-of-line (FEOL) processing of the semiconductor substrate 102 so that the electronic circuitry 130 is integrated on the first side surface 104 of the semiconductor substrate 102.

The electronic circuitry 130 may be configured for receiving the signals from the first electrode 110 and the second electrode 120 in separate portions of the electronic circuitry 130 for separately processing the signals. Thus, the electronic circuitry 130 may comprise dedicated portions for each electrode 110, 120 of the electrode arrangement 100 so as to enable fast processing of the signals. However, it should be realized that the electronic circuitry 130 may process signals in multiplexed manner or that it may combine signals in various ways for processing signals from plural electrodes 110, 120 in the same portion of the electronic circuitry 130.

The electronic circuitry 130 is connected to a connecting layer 140 arranged above the electronic circuitry 130. The connecting layer 140 may comprise metal portions 142 for providing electrical connections in the plane of the connecting layer 140.

The connecting layer 140 may be formed as a back-end-of-line (BEOL) layer arranged above the electronic circuitry 130, which may be connected by vias to the electronic circuitry 130.

As illustrated in Fig. 1, the second electrode 120 may be connected to the electronic circuitry 130. In this regard, the electrode arrangement 100 may comprise a first interconnect 146, which is configured to electrically connect the connecting layer 140 at a first point 144a of a first metal portion 142a to the electronic circuitry 130.

The first interconnect 146 may be a first vertical interconnect, such as a via, which may extend e.g. through a plurality of BEOL layers to connect to the electronic circuitry 130. The first interconnect 146 need not necessarily extend only in a vertical direction, but may rather extend vertically between layers and extend in the plane of one or more intermediate layers to form a connection between the connecting layer 140 and the electronic circuitry 130. Thus, the first interconnect 146 may comprise a plurality of vias which need not necessarily be aligned on top of each other.

The electrode arrangement 100 may further comprise a second interconnect 148, which is configured to provide an electrical connection from the connecting layer 140 to the first side surface 104 of the semiconductor substrate 102. The second interconnect 148 may be configured to electrically connect the connecting layer 140 at a second point 144b of the first metal portion 142a to the first side surface 104 of the semiconductor substrate 102. Since the first interconnect 146 and the second interconnect 148 are connected to different points 144a, 144b of the same metal portion 142a of the connecting layer 140, the first interconnect 146 and the second interconnect 148 are also electrically connected.

The second interconnect 148 may be a second vertical interconnect, such as a via, which may extend e.g. through a plurality of BEOL layers and the FEOL layers of the electronic circuitry 130 to connect to the first side surface 104 of the semiconductor substrate 102. The second interconnect 148 need not necessarily extend only in a vertical direction, but may rather extend vertically between layers and extend in the plane of one or more intermediate layers to form a connection between the connecting layer 140 and the first side surface 104 of the semiconductor substrate 102. Thus, the second interconnect 148 may comprise a plurality of vias which need not necessarily be aligned on top of each other.

The electrode arrangement 100 may further comprise a first through-substrate via (TSV) 150. The semiconductor substrate 102 may be thinned, as will be described later, so as to allow the TSV 150 to extend between the first side surface 104 and the second side surface 106.

The second interconnect 148 may be connected to the first TSV 150 at the first side surface 104 of the semiconductor substrate 102. The first TSV 150 may provide an electrical connection through the semiconductor substrate 102. The first TSV 150 may be connected at the second side surface 106 to the second electrode 120. Alternatively, the first TSV 150 may be connected to the second electrode 120 through an additional interconnect 152, which may extend between the TSV 150 and the electrode 120.

The additional interconnect 152 may be an additional vertical interconnect, such as a via, which may extend e.g. through a plurality of layers arranged below the second side surface 106 of the semiconductor substrate 102. The additional interconnect 152 need not necessarily extend only in a vertical direction, but may rather extend vertically between layers and extend in the plane of one or more intermediate layers to form a connection between the TSV 150 and the second electrode 120. Thus, the additional interconnect 152 may comprise a plurality of vias which need not necessarily be aligned on top of each other.

The second electrode 120 may thus be electrically connected to the electronic circuitry 130 through the additional interconnect 152, the TSV 150, the second interconnect 148, the metal portion 142a of the connecting layer 140 and the first interconnect 146.

The electrode arrangement 100 may further comprise a second TSV 154 and a third TSV 156 through the semiconductor substrate 102. These additional TSVs 154, 156 may be used in connecting the first electrode 110 to the electronic circuitry 130 as will be described below. However, as shown in Fig. 2, the first electrode 110 may alternatively be connected to the electronic circuitry 130 without the connection being provided through the semiconductor substrate 102.

As illustrated in Fig. 1, the first electrode 110 may be connected to the electronic circuitry 130. In this regard, the electrode arrangement 100 may comprise a third interconnect 158, which is configured to electrically connect the connecting layer 140 at a third point 144c of a second metal portion 142b to the electronic circuitry 130.

The second metal portion 142b may be separate from the first metal portion 142a so as to isolate the connection of the first electrode 110 to the electronic circuitry 130 from the connection of the second electrode 120 to the electronic circuitry 130. It should also be realized that the first metal portion 142a and the second metal portion 142b need not necessarily be formed in the same layer or plane of the electrode arrangement 100.

The third interconnect 158 may be a third vertical interconnect, such as a via, which may extend e.g. through a plurality of BEOL layers to connect to the electronic circuitry 130. The third interconnect 158 need not necessarily extend only in a vertical direction, but may rather extend vertically between layers and extend in the plane of one or more intermediate layers to form a connection between the connecting layer 140 and the electronic circuitry 130. Thus, the third interconnect 158 may comprise a plurality of vias which need not necessarily be aligned on top of each other.

The electrode arrangement 100 may further comprise a fourth interconnect 160, which is configured to provide an electrical connection from the connecting layer 140 to the second TSV 154 at the first side surface 104 of the semiconductor substrate 102. The fourth interconnect 160 may be configured to electrically connect the connecting layer 140 at a fourth point 144d of the second metal portion 142b to the second TSV 154. Since the third interconnect 158 and the fourth interconnect 160 are connected to different points 144c, 144d of the same metal portion 142b of the connecting layer 140, the third interconnect 158 and the fourth interconnect 160 are also electrically connected.

The fourth interconnect 160 may be a fourth vertical interconnect, such as a via, which may extend e.g. through a plurality of BEOL layers and the FEOL layers of the electronic circuitry 130 to connect to the second TSV 154. The fourth interconnect 160 need not necessarily extend only in a vertical direction, but may rather extend vertically between layers and extend in the plane of one or more intermediate layers to form a connection between the connecting layer 140 and the second TSV 154. Thus, the fourth interconnect 160 may comprise a plurality of vias which need not necessarily be aligned on top of each other.

The electrode arrangement 100 may further comprise a fifth interconnect 162, which is configured to provide an electrical connection from the first electrode 110 to the third TSV 156 at the first side surface 104 of the semiconductor substrate 102.

The fifth interconnect 162 may be a fifth vertical interconnect, such as a via, which may extend e.g. through a plurality of BEOL layers and the FEOL layers of the electronic circuitry 130 to connect to the first side surface 104 of the semiconductor substrate 102. The fifth interconnect 162 need not necessarily extend only in a vertical direction, but may rather extend vertically between layers and extend in the plane of one or more intermediate layers to form a connection between the connecting layer 140 and the third TSV 156. Thus, the fifth interconnect 162 may comprise a plurality of vias which need not necessarily be aligned on top of each other.

The second TSV 154 and the third TSV 156 may further be connected at the second side surface 106 of the semiconductor substrate 102 through a bridging structure 164. The bridging structure 164 may be configured to extend in a plane at the second side surface 106 of the semiconductor substrate 102 or may also extend vertically in a structure arranged at the second side surface 106 of the semiconductor substrate 102.

The first electrode 110 may thus be electrically connected to the electronic circuitry 130 through the fifth interconnect 162, the third TSV 156, the bridging structure 164, the second TSV 154, the fourth interconnect 160, the metal portion 142b of the connecting layer 140 and the third interconnect 158.

Thanks to the first electrode 110 being connected to the electronic circuitry 130 through the second TSV 154 and the third TSV 156, the first electrode 110 need not be connected to the electronic circuitry 130 when the first electrode 110 is formed, e.g. by plasma etching. Rather, the second TSV 154 and the third TSV 156 may be connected by the bridging structure 164 being provided on a bottom carrier substrate which is bonded to the semiconductor substrate 102 after the plasma etching to form the first electrode 110. This implies that PID of the electronic circuitry 130 by the plasma etching of the first electrode 110 may be avoided.

Further, since the connections between the first electrode 110 and the electronic circuitry 130 and between the second electrode 120 and the electronic circuitry 130 extend at the second side surface 106 of the semiconductor substrate 102, BEOL capacitances may be arranged at the second side surface 106 and need not entirely be arranged at the first side surface 104. This implies that layout space at the first side surface 104 is saved, which may ensure that the electrode arrangement 100 may be very compact.

The portions 142a, 142b of the connecting layer 140, the first interconnect 146, the second interconnect 148, the first TSV 150, the additional interconnect 152, the second TSV 154, the third TSV 156, the third interconnect 158, the fourth interconnect 160, the fifth interconnect 162 and the bridging structure 164 may all be formed in a conducting material, such as a metal. For instance, copper or aluminum may be used.

The connections may be surrounded by a dielectric material to isolate the connections. For instance, interlevel dielectric oxide 166 may be provided to isolate between different connections in the electrode arrangement 100.

The TSVs 150, 154, 156 may be formed as structures extending into the first side surface 104 of the semiconductor substrate 102. Side walls of the structures may be provided with a TSV liner to isolate the metal of the TSV 150, 154, 156 from the semiconductor substrate 102. The semiconductor substrate 102 may then be thinned from the second side surface 106 such that the TSVs 150, 154, 156 are exposed at the second side surface 106 and extend through the semiconductor substrate 102.

Typically, the thinned semiconductor substrate 102 may be 50 µm thick. A cross-section of the TSVs 150, 154, 156 at the first side surface 104 may have a dimension of 5 µm, which may be appropriate to ensure that the TSV 150, 154, 156 may extend through the thickness of the semiconductor substrate 102.

A distance between the fourth interconnect 160 and the fifth interconnect 162 may for instance be 10 µm, which may ensure that the first electrode 110 is isolated from the electronic circuitry 130 when the first electrode 110 is formed. This may also be appropriate to ensure that the bridging structure 164 need not be very large. However, it should be realized that the relations between the fifth interconnect 162 and the fourth interconnect 160 may be completely different. For instance, the third interconnect 158 may even be arranged between the fifth interconnect 162 and the fourth interconnect 160.

The first and second electrodes 110, 120 may each have a diameter of 25 µm. This may be suitable for recording of electrical signals, e.g. when recoding electrical signals in biological matter, such as a brain.

Each pair of a first electrode 110 above the first side surface 104 and a second electrode 120 below the second side surface 106 may be connected in an identical manner to the electronic circuitry 130 as described above. The electrode arrangement 100 including the electronic circuitry 130 associated both with the first electrode 110 and the second electrode 120 may thus be arranged in a regular manner having an electrode pitch of 35 µm. Thus, the electrodes 110, 120 in the first and the second array may be arranged very close to each other.

The above dimensions of TSVs 150, 154, 156, distances between interconnects and dimensions of electrodes 110, 120 should be seen as examples and is in no way limiting on the electrode arrangement 100 of the present application. As understood by the person skilled in the art, other dimensions may be used.

Fig. 2 illustrates an electrode arrangement 200 according to a second embodiment. Similar to the electrode arrangement 100 of the first embodiment, the electrode arrangement 200 of the second embodiment comprises a semiconductor carrier substrate 202, having a first side surface 204 and a second side surface 206 opposite to the first side surface 204, a first electrode 210 in a first array above the first side surface 204 and a second electrode 220 in a second array below the second side surface 206, the first and the second electrodes 210, 220 being connected to electronic circuitry 230 arranged above the first side surface 204.

The second electrode 220 may be connected to the electronic circuitry 230 in a corresponding manner as described above for the electrode arrangement 100 of the first embodiment through an additional interconnect 252, a first TSV 250, a second interconnect 248, a metal portion 242a of the connecting layer 240 and a first interconnect 246 and this connection will not be further described in relation to Fig. 2.

As illustrated in Fig. 2, the first electrode 210 may be connected to the electronic circuitry 230 in a different manner than the connection described above for the electrode arrangement 100. In this regard, the electrode arrangement 200 may comprise a third interconnect 254, which is configured to electrically connect the connecting layer 240 at a third point 244c of a second metal portion 242b to the electronic circuitry 230.

The second metal portion 242b may be separate from the first metal portion 242a so as to isolate the connection of the first electrode 210 to the electronic circuitry 230 from the connection of the second electrode 220 to the electronic circuitry 230. It should also be realized that the first metal portion 242a and the second metal portion 242b need not necessarily be formed in the same layer or plane of the electrode arrangement 200.

The third interconnect 254 may be a third vertical interconnect, such as a via, which may extend e.g. through a plurality of BEOL layers to connect to the electronic circuitry 230. The third interconnect 254 need not necessarily extend only in a vertical direction, but may rather extend vertically between layers and extend in the plane of one or more intermediate layers to form a connection between the connecting layer 240 and the electronic circuitry 230. Thus, the third interconnect 254 may comprise a plurality of vias which need not necessarily be aligned on top of each other.

The electrode arrangement 200 may further comprise a fourth interconnect 256, which is configured to provide an electrical connection from the first electrode 210 to the connecting layer 240. The fourth interconnect 256 may be configured to electrically connect the connecting layer 240 at a fourth point 244d of the second metal portion 242b to the first electrode 210. Since the third interconnect 254 and the fourth interconnect 256 are connected to different points 244c, 244d of the same metal portion 242b of the connecting layer 240, the third interconnect 254 and the fourth interconnect 256 are also electrically connected.

The fourth interconnect 256 may be a fourth vertical interconnect, such as a via, which may extend e.g. through a plurality of BEOL layers to connect to the electrode 210. The fourth interconnect 256 need not necessarily extend only in a vertical direction, but may rather extend vertically between layers and extend in the plane of one or more intermediate layers to form a connection between the connecting layer 240 and the first electrode 210. Thus, the fourth interconnect 256 may comprise a plurality of vias which need not necessarily be aligned on top of each other.

The first electrode 210 may thus be electrically connected to the electronic circuitry 230 through the fourth interconnect 256, the metal portion 242b of the connecting layer 240 and the third interconnect 254.

As will be described later, the first electrode 210 may be formed on a top carrier substrate which is later bonded to the semiconductor substrate 102 such that the first electrode 210 need not be connected to the electronic circuitry 230 when the first electrode 210 is formed, e.g. by plasma etching. This implies that PID of the electronic circuitry 230 by the plasma etching of the first electrode 210 may be avoided.

According to an alternative, PID issues may be resolved by adding electrostatic discharge protection to the first electrode 210 such that the first electrode 210 may be formed while connected to the electronic circuitry 230.

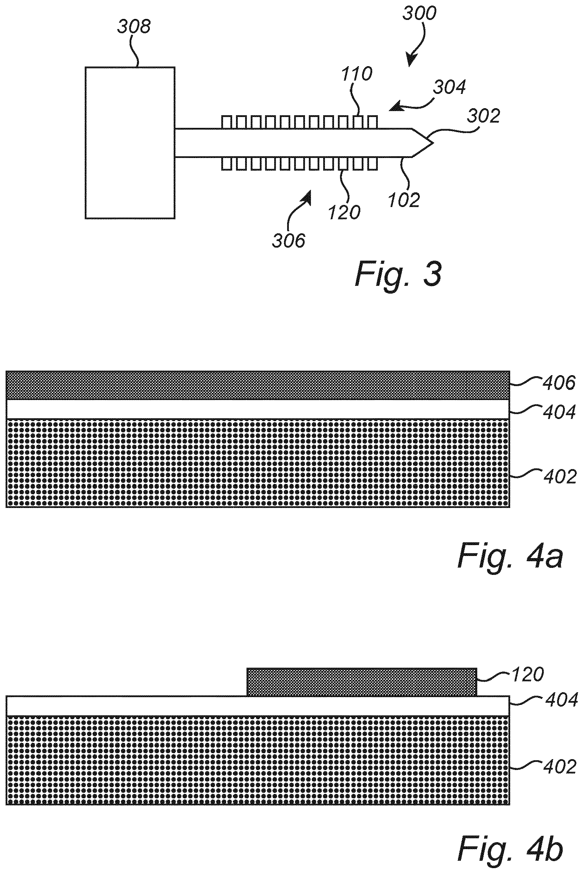

Referring now to Fig. 3, the electrode arrangement 100 according to the first embodiment or the electrode arrangement 200 according to the second embodiment may be arranged in a neural probe 300. For brevity, reference is made below only to the electrode arrangement 100 according to the first embodiment.

The semiconductor substrate 102 may be adapted for being inserted into a brain. The semiconductor substrate 102 may comprise a pointed tip 302 for facilitating insertion of the neural probe into the brain.

The semiconductor substrate 102 may further be elongate and thin in order to have a small impact on the brain. A first array 304 of first electrodes 110 may be arranged on a front side of the thin semiconductor substrate 102 and a second array 306 of second electrodes 120 may be arranged on a back side of the thin semiconductor substrate 102.

The electronic circuitry 130 may be connected by one or more wires to a base portion 308 of the neural probe 300 for transferring processed signals to the base portion 308. The wires may be dedicated to a portion of the electronic circuitry 130 associated with a single electrode. Alternatively, a wire may be associated with plural portions of the electronic circuitry 130, e.g. for transferring signals in a multiplexed manner.

The base portion 308 may comprise a processing unit for refining information in the signals recorded by the first array 304 of first electrodes 110 and the second array 306 of second electrodes 120. However, the base portion 308 may alternatively be connected to an external unit, e.g. using wired or wireless communication, for transferring signals to the external unit for further processing therein.

Referring now to Figs 4a-n, a method according to a first embodiment for manufacturing of an electrode arrangement will be described. The method according to the first embodiment may be used for manufacturing an electrode arrangement 100 according to the first embodiment described above.

As shown in Fig. 4a, a bottom carrier substrate 402 may be processed. The bottom carrier substrate 402 may comprise a semiconductor wafer 402 which is provided with a planar oxide layer 404 on the entire semiconductor wafer 402.

As further shown in Fig. 4a, processing of the bottom carrier substrate 402 may comprise depositing a layer 406 of a material that is to be used for the second array of electrodes 120 on the oxide layer 404. For instance, a TiN layer 406 may be deposited, e.g. by sputtering deposition.

As shown in Fig. 4b, processing of the bottom carrier substrate 402 may further comprise etching the TiN layer 406 so as to form the second array of electrodes 120. The etching of the TiN layer 406 may be performed using plasma etching, e.g. by reactive-ion etching.

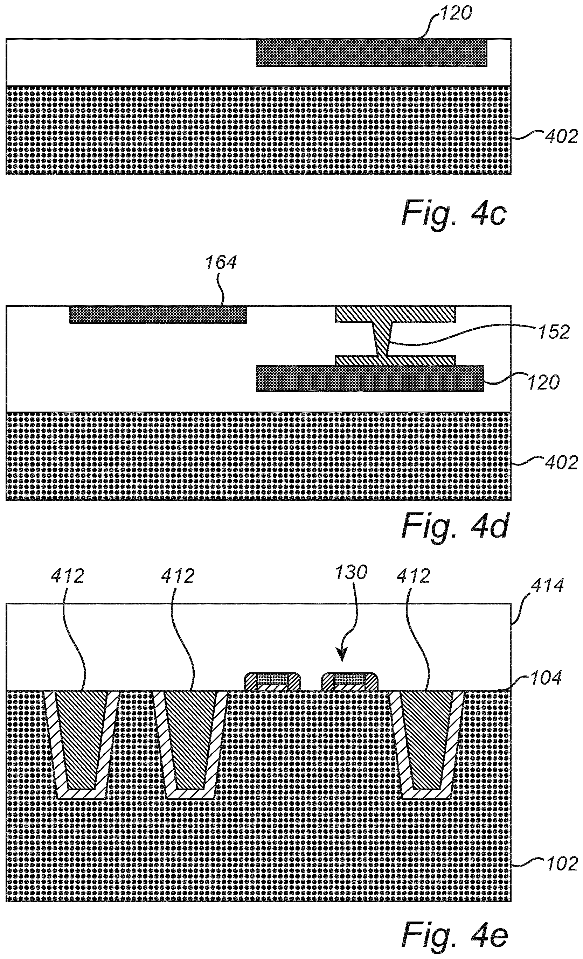

As shown in Fig. 4c, oxide deposition may be performed on the bottom carrier substrate 402, followed by planarization of the oxide surface and an etch, e.g. chemical-mechanical polishing (CMP), stopping on the TiN layer 406 to form a planar top surface on the bottom carrier substrate 402 exposing the TiN electrodes 120.

As shown in Fig. 4d, the additional interconnect 152 being connected to the electrode 120 may be formed by BEOL processing, e.g. using damascene technology, on the bottom carrier substrate 402, exposing a metal contact at the top surface of the bottom carrier substrate 402. Also, a bridging structure 164 may be formed at the top surface of the bottom carrier substrate 402. The top surface of the bottom carrier substrate 402 may thus comprise metal portions as well as oxide portions.

Referring now to Fig. 4e, processing of a semiconductor carrier substrate 102 is described. The semiconductor substrate 102 may be a silicon wafer.

The processing of the semiconductor substrate 102 may include forming an electronic circuitry 130 on the first side surface 104 of the semiconductor substrate 102. Also, a plurality of structures 412 extending from the first side surface 104 into the semiconductor substrate 102 may be formed. These structures 412 may be filled with a thin dielectric TSV liner on sidewalls and bottom of the structures 412 and then be filled by a conducting material, e.g. a metal. Thus, metal-filled structures 412 may be formed.

Then, as shown in Fig. 4e, an oxide layer 414 may be deposited on the semiconductor substrate 102 and planarized.

The forming of the electronic circuitry 130 and the metal-filled structures 412 may be achieved by standard FEOL processing and processing to form TSVs.

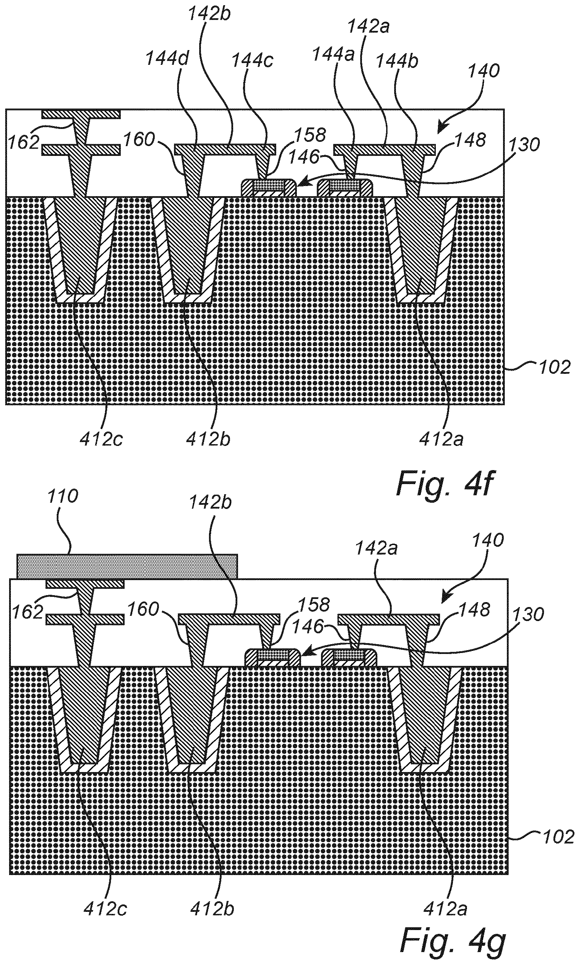

As shown in Fig. 4f, connections may then be formed to the metal-filled structures 412 and the electronic circuitry 130. Such connections may be formed e.g. by standard BEOL processing.

Thus, a connecting layer 140 comprising the first metal portion 142a and the second metal portion 142b may be formed. Further, a first interconnect 146 connecting the first metal portion 142a to the electronic circuitry 130, a second interconnect 148 connecting the first metal portion 142a to a first metal-filled structure 412a, a third interconnect 158 connecting the second metal portion 142b to the electronic circuitry 130, a fourth interconnect 160 connecting the second metal portion 142b to a second metal-filled structure 412b, and a fifth interconnect 162 connecting a top surface above the semiconductor substrate 102 to ta third metal-filled structure 412c may be formed.

As shown in Fig. 4g, a first electrode 110 may then be formed on a top surface above the semiconductor substrate 102 in contact with the fifth interconnect 162. The forming of the first electrode 110 may be performed by deposition and etching of TiN in a corresponding manner as described above for forming of the second electrode 120.

It should be noted that, as shown in Fig. 4g, the first electrode 110 is not connected to the electronic circuitry 130 during etching of the first electrode 110 since the second metal-filled structure 412b and the third metal-filled structure 412c are not connected. Thus, PID of the electronic circuitry 130 due to plasma etching of the first electrode 110 may be avoided.

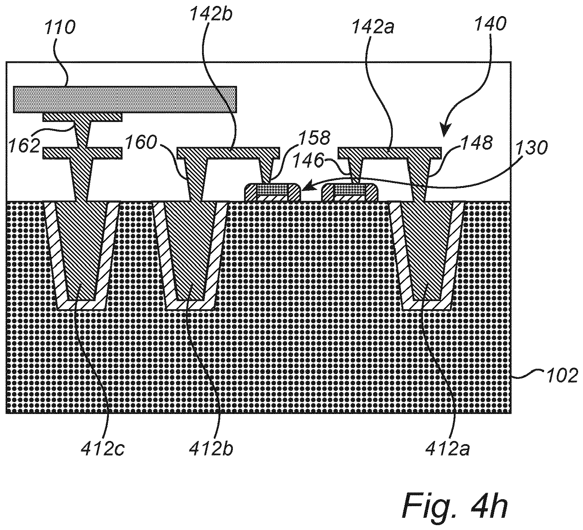

As shown in Fig. 4h, the first array of first electrodes 110 may then be encapsulated in dielectric material and a surface may be planarized to prepare the semiconductor substrate 102 for bonding.

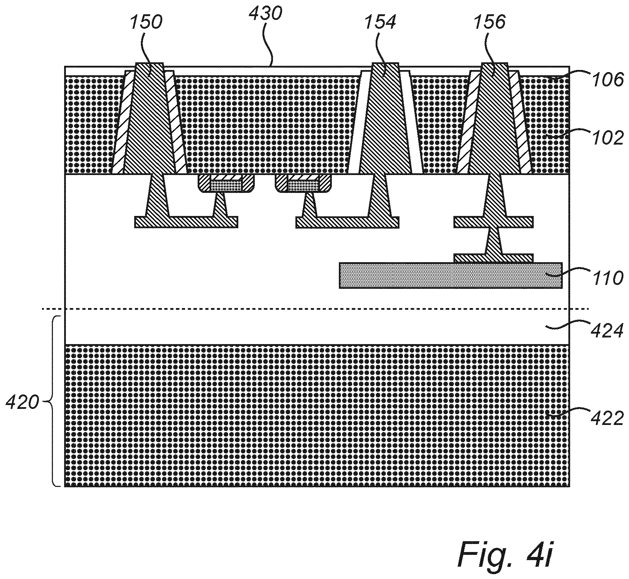

As shown in Fig. 4i, the semiconductor substrate 102 may then be flipped and bonded to a top carrier substrate 420, e.g. by dielectric bonding, or by a temporary bonding technique, such as using an adhesive. If temporary bonding is used, the top carrier substrate 420 may even be reused, e.g. in manufacturing of different electrode arrangements. The bonding interface is indicated in Fig. 4i as a dashed line. The top carrier substrate 420 may thus be a blank semiconductor wafer 422 (or a glass carrier wafer if temporary bonding is used) with a planar oxide surface 424 extending over the entire area of the semiconductor wafer 422.

As further shown in Fig. 4i, after bonding of the top carrier substrate 420 to the semiconductor substrate 102, the semiconductor substrate 102 may be thinned from the second side surface 106, e.g. by grinding or CMP. The thinning of the semiconductor substrate 102 reveals the metal-filled structures 412 such that these will form TSVs 150, 154, 156, respectively, through the semiconductor substrate 102. Further, after thinning of the semiconductor substrate 102, an oxide layer 430 may be formed at the second side surface 106 of the semiconductor substrate 102. This implies that an isolation on the semiconductor substrate 102 will be formed. The TSVs 150, 154, 156 are arranged to extend through the oxide layer 430.

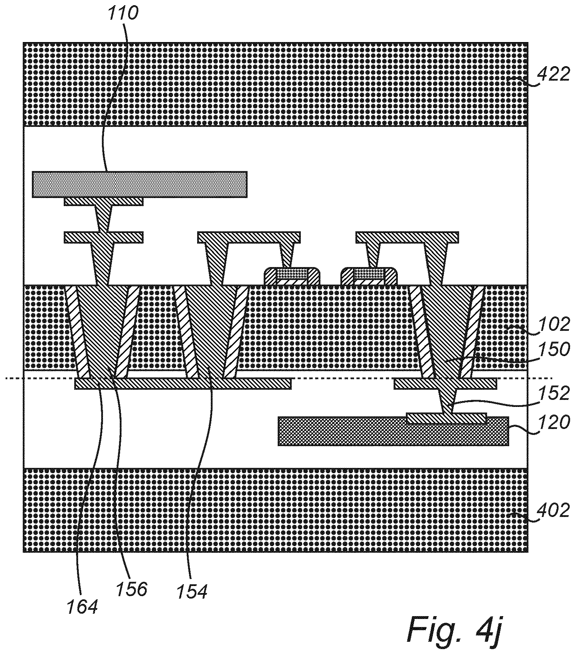

As shown in Fig. 4j, the semiconductor substrate 102 may then be bonded to the bottom carrier substrate 402 illustrated in Fig. 4e. The bonding interface is indicated in Fig. 4j as a dashed line. The bonding of the semiconductor substrate 102 and the bottom carrier substrate 402 connects the second electrode 120 to the first TSV 150 so as to connect the second electrode 120 to the electronic circuitry 130. Further, the bonding of the semiconductor substrate 102 and the bottom carrier substrate 402 connects the bridging structure 164 to the second TSV 154 and the third TSV 156 so as to connect the first electrode 110 to the electronic circuitry 130.

Then, processing is performed to reveal the first array of first electrodes 110 and the second array of second electrodes 120.

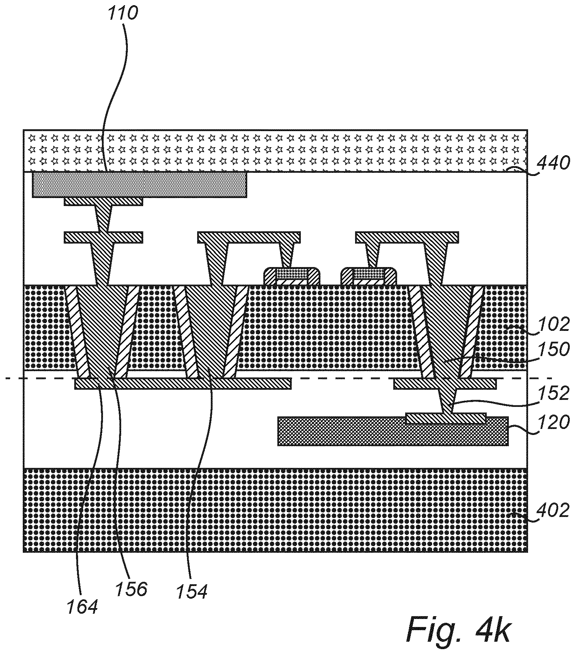

As shown in Fig. 4k, the top carrier substrate 420 may first be removed and the oxide layer above the first electrode 110 may be removed. This may be performed by various methods of material removal, e.g. by a combination of grinding, CMP, plasma etch, and wet etch to remove the top carrier substrate 420, or debonding of the top carrier substrate 420 if a temporary bonding is used for removing the top carrier substrate 420, and CMP stopping on TiN to remove the oxide layer 424 above the first electrode 110. Thus, a front surface 440 of the electrode arrangement 100 may be defined, wherein the first array of first electrodes 110 is exposed.

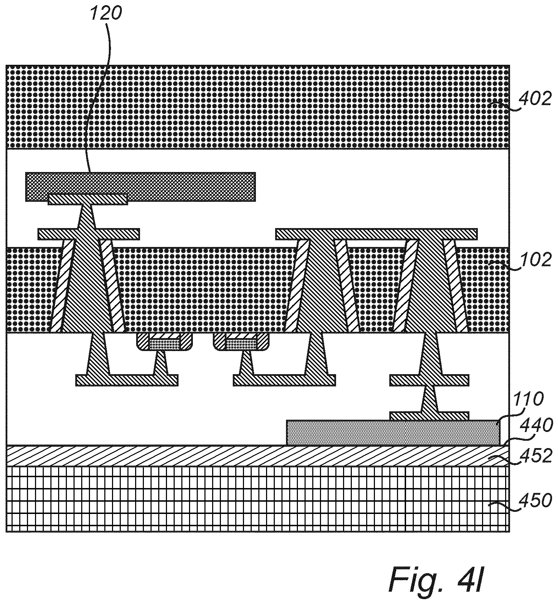

As shown in Fig. 4l, the semiconductor substrate 102 may then be flipped and the front surface 440 may be bonded to a temporary wafer 450, which will allow removal of the bottom carrier substrate 402.

The front surface 440 may be attached to the temporary wafer 450 e.g. by an adhesive layer 452 providing an attachment that may be easily removed while still providing sufficient strength to enable processing of the electrode arrangement 100 attached to the temporary wafer 450.

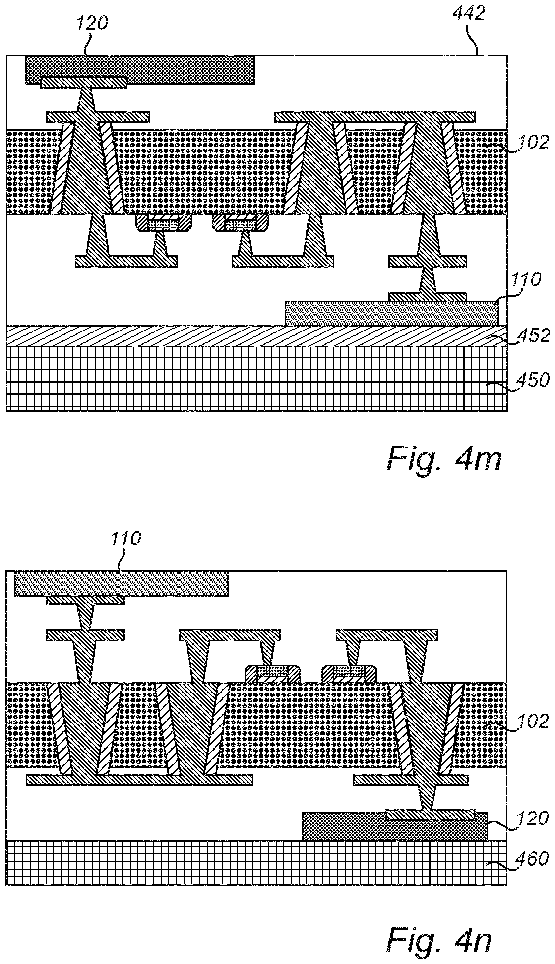

As shown in Fig. 4m, the bottom carrier substrate 402 and the oxide layer 404 on the bottom carrier substrate 402 may then be removed. This may be performed by various methods of material removal, e.g. by a combination of grinding, CMP, and plasma etch to remove the bottom carrier substrate 402 and CMP stopping on TiN to remove the oxide layer 404 to reveal the second electrode 120. Thus, a back surface 442 of the electrode arrangement 100 may be defined, wherein the second array of second electrodes 120 is exposed.

As shown in Fig. 4n, the semiconductor substrate 102 may then be flipped again, the back surface 442 of the electrode arrangement 100 may be bonded to a dicing tape 460 and the temporary wafer 450 may be removed. The electrode arrangement 100 is thus complete and may be transferred to dicing and packaging.

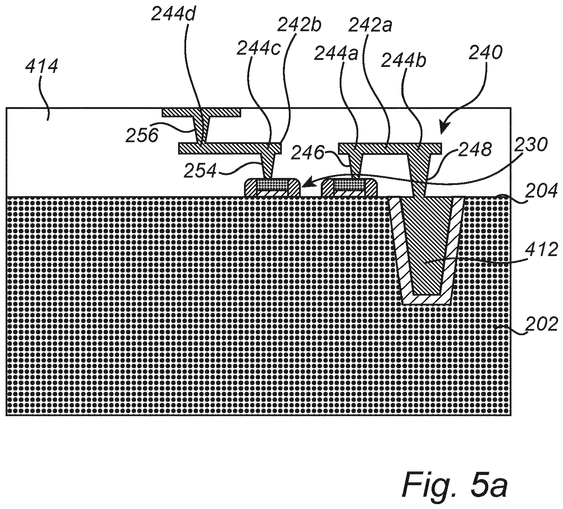

Referring now to Figs 5a-b, a method according to a second embodiment for manufacturing of an electrode arrangement will be described. The method according to the second embodiment may be used for manufacturing an electrode arrangement 200 according to the second embodiment described above.

The method according to the second embodiment may include processing of a bottom carrier substrate 402 in the same manner as described above in the method according to the first embodiment to form the second array of electrodes 220 and the additional interconnect 252. However, the bridging structure 164 need not be formed at the top surface of the bottom carrier substrate 402.

Referring now to Fig. 5a, processing of a semiconductor carrier substrate 202 is described. The semiconductor substrate 202 may be a silicon wafer.

The processing of the semiconductor substrate 202 may include forming an electronic circuitry 230 on the first side surface 204 of the semiconductor substrate 202. Also, a structure 412 extending from the first side surface 204 into the semiconductor substrate 202 may be formed. This structure 412 may be filled with a thin dielectric TSV liner on sidewalls and bottom of the structures 412 and then be filled by a conducting material, e.g. a metal. Thus, metal-filled structures 412 may be formed.

Then, as shown in Fig. 5a, an oxide layer 414 may be deposited on the semiconductor substrate 102 and planarized.

The forming of the electronic circuitry 230 and the metal-filled structure 412 may be achieved by standard FEOL processing and processing to form TSVs.

As further shown in Fig. 5a, connections may then be formed to the metal-filled structures 412 and the electronic circuitry 230. Such connections may be formed e.g. by standard BEOL processing.

Thus, a connecting layer 240 comprising the first metal portion 242a and the second metal portion 242b may be formed. Further, a first interconnect 246 connecting the first metal portion 242a to the electronic circuitry 230, a second interconnect 248 connecting the first metal portion 242a to the metal-filled structure 412, a third interconnect 254 connecting the second metal portion 242b to the electronic circuitry 230, and a fourth interconnect 256 connecting the second metal portion 242b to a top surface above the semiconductor substrate 202 may be formed.

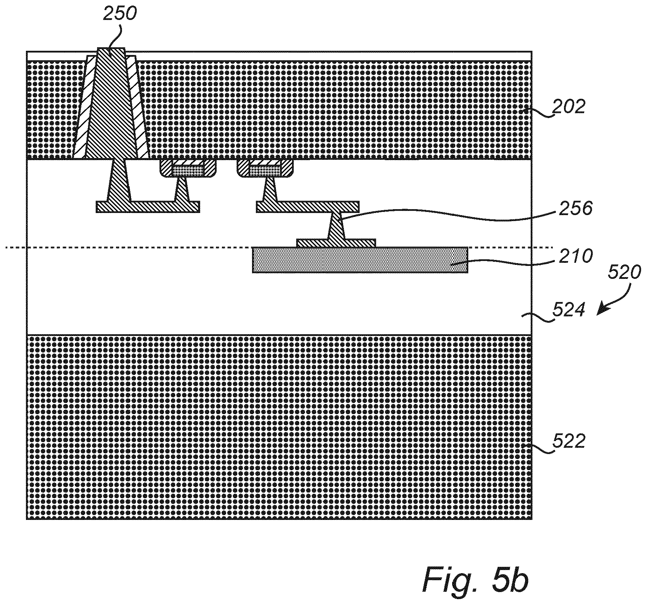

As shown in Fig. 5b, the semiconductor substrate 202 may then be flipped and bonded, e.g. by hybrid bonding, to a top carrier substrate 520 which has been processed before the bonding. The bonding interface is indicated in Fig. 5b as a dashed line.

The top carrier substrate 520 may comprise a semiconductor wafer 522 with a planar oxide surface 524 on which the first array of electrodes 210 have been formed, in a similar manner as described above for forming the second array of electrodes 120 with reference to Figs 4a-c. The bonding of the top carrier substrate 520 to the semiconductor substrate 202 may thus connect the first electrode 210 to the fourth interconnect 256.

It should be noted that the first electrode 210 is not connected to the electronic circuitry 230 during etching of the first electrode 210 since the first electrode 210 is formed on the top carrier substrate 520. Thus, PID of the electronic circuitry 130 due to plasma etching of the first electrode 110 may be avoided.

Once the top carrier substrate 520 has been bonded to the semiconductor substrate 202, the method may proceed with steps illustrated and discussed in Figs 4i-n above in order to complete manufacturing of the electrode arrangement 200.

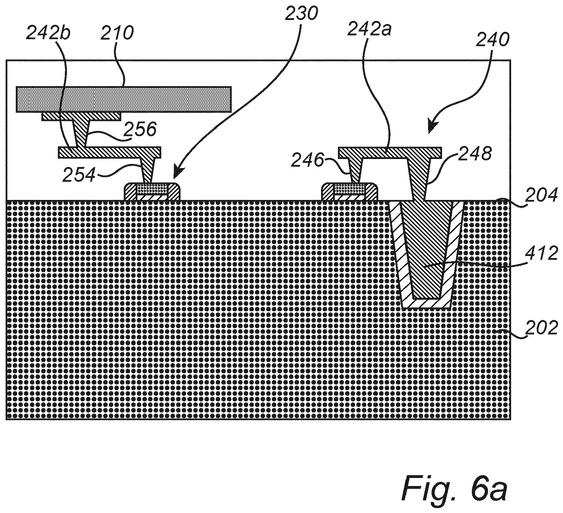

Referring now to Figs 6a-f, a method according to a third embodiment for manufacturing of an electrode arrangement will be described. The method according to the third embodiment may use forming of electrodes while being connected to the electronic circuitry such that PID issues may need to be handled in a different manner. For instance, PID issues may be resolved by adding electrostatic discharge protection to the electrodes.

The final structure of the electrode arrangement manufactured by the method according to the third embodiment may be similar to the electrode arrangement 200 described above.

Referring now to Fig. 6a, processing of a semiconductor carrier substrate 202 is described. The semiconductor substrate 202 may be a silicon wafer.

The processing of the semiconductor substrate 202 may include forming an electronic circuitry 230 on the first side surface 204 of the semiconductor substrate 202. Also, a structure 412 extending from the first side surface 204 into the semiconductor substrate 202 may be formed. This structure 412 may be filled with a thin dielectric TSV liner on sidewalls and bottom of the structures 412 and then be filled by a conducting material, e.g. a metal. Thus, metal-filled structures 412 may be formed.

The forming of the electronic circuitry 230 and the metal-filled structure 412 may be achieved by standard FEOL processing and processing to form TSVs.

As further shown in Fig. 6a, connections may then be formed to the metal-filled structures 412 and the electronic circuitry 230. Such connections may be formed e.g. by standard BEOL processing.

Thus, a connecting layer 240 comprising the first metal portion 242a and the second metal portion 242b may be formed. Further, a first interconnect 246 connecting the first metal portion 242a to the electronic circuitry 230, a second interconnect 248 connecting the first metal portion 242a to the metal-filled structure 412, a third interconnect 254 connecting the second metal portion 242b to the electronic circuitry 230, and a fourth interconnect 256 connecting the second metal portion 242b to a top surface above the semiconductor substrate 202 may be formed.

Further, the first electrode 110 may be directly processed on the top surface to form the first electrode 110 connected to the fourth interconnect 256.

Then, the first array of first electrodes 110 may be encapsulated in dielectric material and a surface may be planarized to prepare the semiconductor substrate 102 for bonding.

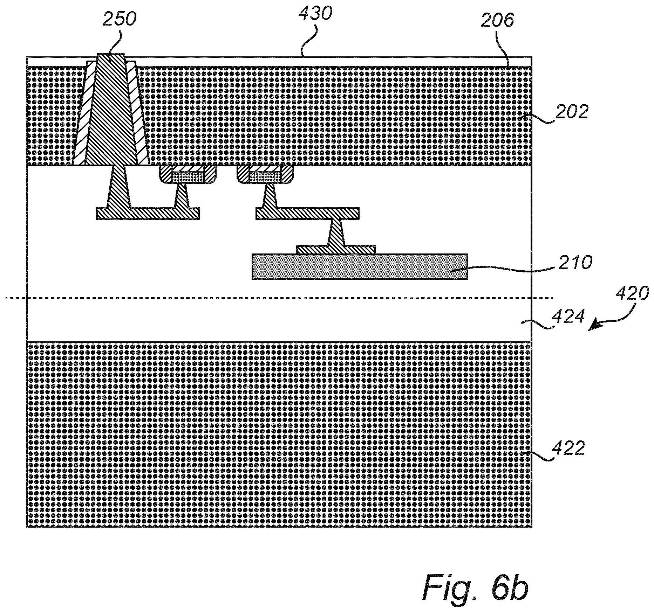

As shown in Fig. 6b, the semiconductor substrate 102 may then be flipped and bonded to a top carrier substrate 420, e.g. by dielectric bonding. The bonding interface is indicated in Fig. 6b as a dashed line. The top carrier substrate 420 may thus be a blank semiconductor wafer 422 with a planar oxide surface 424 extending over the entire area of the semiconductor wafer 422.

As further shown in Fig. 6b, after bonding of the top carrier substrate 420 to the semiconductor substrate 202, the semiconductor substrate 202 may be thinned from the second side surface 206, e.g. by grinding or CMP. The thinning of the semiconductor substrate 202 reveals the metal-filled structures 412 such a TSV 250 will be formed through the semiconductor substrate 202. Further, after thinning of the semiconductor substrate 202, an oxide layer 430 may be formed at the second side surface 206 of the semiconductor substrate 202. This implies that an isolation on the semiconductor substrate 202 will be formed. The TSV 250 is arranged to extend through the oxide layer 430.

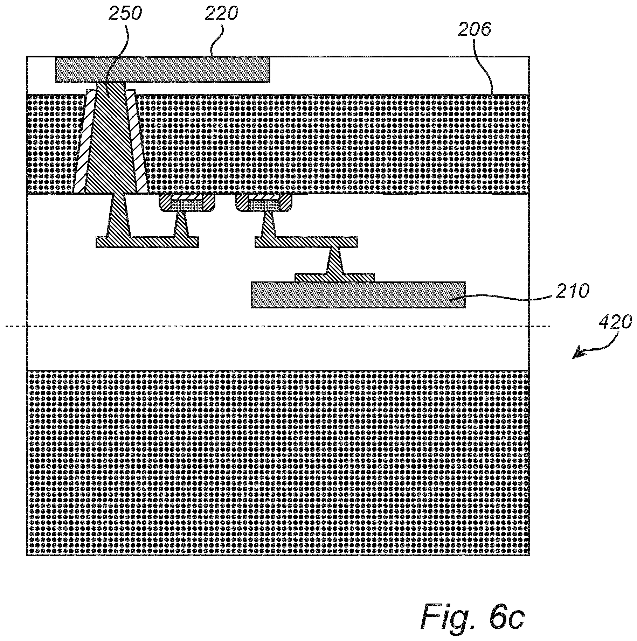

As shown in Fig. 6c, the second array of electrodes 220 may then be directly processed at the second side surface 206 to form the second electrode 220 connected to the TSV 250. Then, oxide deposition may be performed at the second side surface 206, followed by planarization of the oxide surface and an etch, e.g. chemical-mechanical polishing (CMP), stopping on the TiN layer to form a planar back side of the electrode arrangement 200 exposing the TiN electrodes 220.

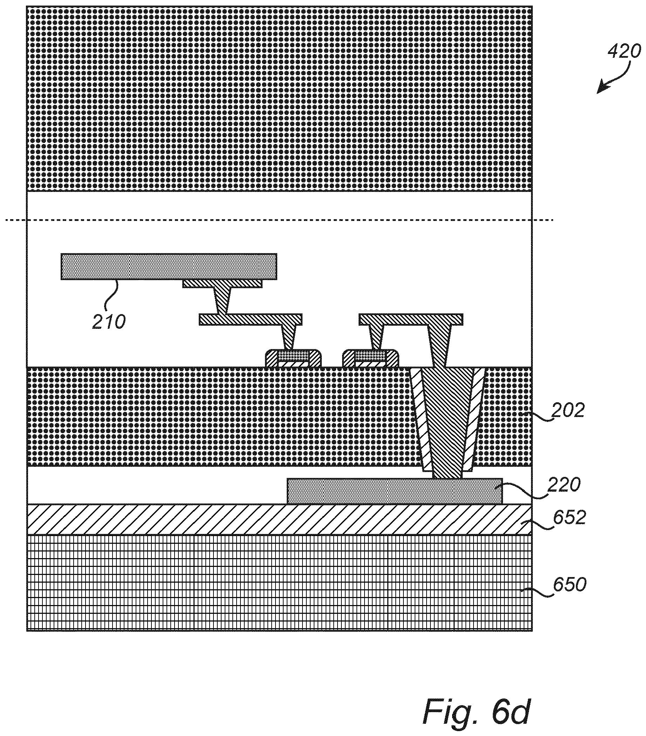

As shown in Fig. 6d, the semiconductor substrate 202 may then be flipped and the back surface may be bonded to a temporary wafer 650, which will allow removal of the top carrier substrate 420.

The back surface may be attached to the temporary wafer e.g. by an adhesive layer 652 providing an attachment that may be easily removed while still providing sufficient strength to enable processing of the electrode arrangement 200 attached to the temporary wafer 650.

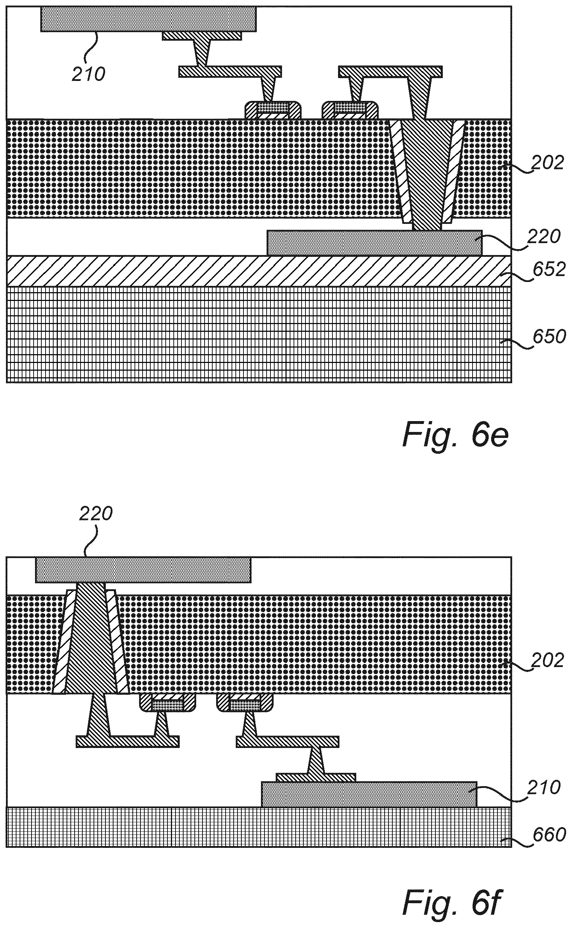

As shown in Fig. 6e, the semiconductor wafer 422 and the oxide layer 424 may then be removed. This may be performed by various methods of material removal, e.g. by a combination of grinding, CMP, and plasma etch to remove the semiconductor wafer 422 and CMP stopping on TiN to remove the oxide layer 424 to reveal the first electrode 210. Thus, a front surface of the electrode arrangement 200 may be defined, wherein the first array of first electrodes 210 is exposed.

As shown in Fig. 6f, the semiconductor substrate 202 may then be flipped again, the front surface of the electrode arrangement 200 may be bonded to a dicing tape 660 and the temporary wafer 650 may be removed. The electrode arrangement 200 is thus complete and may be transferred to dicing and packaging.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. An electrode arrangement (100; 200), comprising:
a semiconductor carrier substrate (102; 202) having a first side surface (104; 204) and a second side surface (106; 206) opposite to the first side surface (104; 204);
a first array of electrodes (110; 210) arranged above the first side surface (104; 204);
a second array of electrodes (120; 220) arranged below the second side surface (106; 206);
an electronic circuitry (130; 230) for processing electrical signals recorded by the first array of the electrodes (110; 210) and the second array of electrodes (120; 220), said electronic circuitry (130; 230) comprising at least one layer arranged above the first side surface (104; 204) and below the first array of electrodes (110; 210);
a connecting layer (140 240) arranged above the at least one layer of the electronic circuitry (130; 230), said connecting layer (140; 240) being configured to connect the electrodes (110; 210) of the first array and the electrodes (120; 220) of the second array to the electronic circuitry (130; 230), wherein the connecting layer (140; 240) is configured to provide a first connection (142a; 242a) in a plane of the connecting layer (140; 240) between a first point (144a; 244a) and a second point (144b; 244b) for connecting an electrode (120; 220) in the second array to the electronic circuitry (130; 230);
a first interconnect (146; 246) for electrically connecting the first point (144a; 244a) to the electronic circuitry (130; 230);
a plurality of through-substrate vias, TSVs, (150; 250) extending through the semiconductor carrier substrate (102; 202) between the first side surface (104; 204) and the second side surface (106; 206) for forming an electrical connection through the semiconductor carrier substrate (102; 202);
a second interconnect (148; 248) extending through a plane defined by the at least one layer of the electronic circuitry (130; 230), wherein the second interconnect (148; 248) and a first TSV (150; 250) of the plurality of TSVs electrically connect the second point (144b; 244b) to the electrode (120; 220) in the second array.

2. The electrode arrangement according to claim 1, wherein the connecting layer (140) is configured to provide a second connection (142b) in a plane of the connecting layer (140) between a third point (144c) and a fourth point (144d) for connecting an electrode (110) in the first array to the electronic circuitry (130), the electrode arrangement (100) further comprising a third interconnect (158) for electrically connecting the third point (144c) to the electronic circuitry (130), a fourth interconnect (160) for connecting the fourth point (144d) to a second TSV (154) of the plurality of TSVs, a fifth interconnect (162) for connecting the electrode (110) in the first array to a third TSV (156) of the plurality of TSVs, and a bridging structure (164) arranged at the second side surface (106) for connecting the second TSV (154) to the third TSV (156).

3. The electrode arrangement according to claim 1, wherein the connecting layer (240) is configured to provide a second connection (242b) in a plane of the connecting layer (240) between a third point (244c) and a fourth point (244d) for connecting an electrode (220) in the first array to the electronic circuitry (230), the electrode arrangement (200) further comprising a third interconnect (254) for electrically connecting the third point (244c) to the electronic circuitry (230), and a fourth interconnect (256) for connecting the electrode (220) in the first array to the fourth point (244d).

4. The electrode arrangement according to any one of the preceding claims, further comprising a back-end-of-line capacitance structure formed at the second side surface (106; 206) between the second array of electrodes (120; 220) and the semiconductor carrier substrate (102; 202).

5. A neural probe, said neural probe (300) comprising the electrode arrangement (100; 200) according to any one of the preceding claims, wherein the semiconductor carrier substrate (102; 202) is adapted for being inserted into a brain.

6. A method for manufacturing an electrode arrangement, said method comprising:
forming an electronic circuitry (130; 230) above a first side surface (104; 204) of a semiconductor carrier substrate (102; 202), said electronic circuitry (130; 230) comprising at least one layer;
forming a plurality of metal-filled structures (412) extending from the first side surface (104; 204) into the semiconductor carrier substrate (102; 202);
forming a connecting layer (140 240) above the electronic circuitry (130; 230) and forming interconnects between the connecting layer (140; 240) and the electronic circuitry (130; 230) and between the connecting layer (140; 240) and the metal-filled structures (412) such that the connecting layer (140; 240) is configured to provide a first connection (142a; 242a) in a plane of the connecting layer (140; 240) between a first point (144a; 244a) connected by a first interconnect (146; 246) to the electronic circuitry (130; 230) and a second point (144b; 244b) connected by a second interconnect (148; 248), extending through a plane defined by the at least one layer of the electronic circuitry (130; 230), to a first metal-filled structure (412a) of the plurality of metal-filled structures (412);
forming a first array of electrodes (110; 210) above the electronic circuitry (130; 230);
thinning the semiconductor carrier substrate (102; 202) to define a second side surface (106; 206) of the semiconductor carrier substrate (102; 202) opposite to the first side surface (104; 204) and to expose a bottom of the metal-filled structures (412) so that the metal-filled structures (412) form a plurality of through-substrate vias, TSVs, (150; 250) extending through the semiconductor carrier substrate (102; 202);
forming a second array of electrodes (120; 220) at the second side surface (106; 206) of the semiconductor carrier substrate (102; 202), wherein an electrode (110; 210) of the second array of electrodes (120; 220) is electrically connected by a first TSV (150; 250) of the plurality of TSVs and the second interconnect (148; 248) to the second point (144b; 244b) in the connecting layer (140; 240).

7. The method according to claim 6, wherein said forming of the second array of electrodes (120; 220) comprises:
processing a bottom carrier substrate (402), wherein said processing comprises forming the second array of electrodes (120; 220) on the bottom carrier substrate (402) and defining electrical connection structures at a surface on the bottom carrier substrate, each electrical connection structure being arranged at or being electrically connected to an electrode (120; 220) in the second array of electrodes (120; 220); and
bonding the surface of the bottom carrier substrate (402) to a second side surface (106; 206) of the semiconductor carrier substrate (102; 202) by hybrid bonding.

8. The method according to claim 7, wherein forming the connecting layer (140) and forming interconnects comprises forming the connecting layer (140) to provide a second connection (142b) in a plane of the connecting layer (140) between a third point (144c) connected by a third interconnect (158) to the electronic circuitry (130) and a fourth point (144d) connected by a fourth interconnect (160) to a second metal-filled structure (412b) of the plurality of metal-filled structures, wherein the method further comprises forming a fifth interconnect (162) for connecting an electrode (110) in the first array of electrodes (110) to a third metal-filled structure (412c) of the plurality of metal-filled structures.

9. The method according to claim 8, wherein said processing on the bottom carrier substrate (402) comprises defining an electrical bridging connection structure (164) at a surface on the bottom carrier substrate (402), wherein said electrical bridging connection structure (164) when the surface on the bottom carrier substrate (402) is bonded to the second side surface (106) of the semiconductor carrier substrate (102) forms an electrical connection between a second TSV (154) and a third TSV (156), which second and third TSVs (154, 156) are formed by the second and third metal-filled structures (412b, 412c), respectively.

10. The method according to claim 7, wherein said forming of the first array of electrodes (210) comprises:
processing a top carrier substrate (520), wherein said processing comprises forming the first array of electrodes (210) on the top carrier substrate (520), each electrode (210) in the first array of electrodes (210) being arranged at or being electrically connected to an electrical connection structure at a surface on the top carrier substrate (520); and
bonding the surface on the top carrier substrate (520) to a top surface above the first side surface (206) of the semiconductor carrier substrate (202) by hybrid bonding.

11. The method according to any one of claims 6-9, further comprising forming a planar oxide surface on the first array of electrodes (110; 210) on the semiconductor carrier substrate (102; 202) and bonding an oxide surface (424) on a top carrier substrate (420) to the planar oxide surface of the semiconductor carrier substrate (102; 202) by dielectric bonding.

12. The method according to claim 10 or 11, further comprising after said thinning of the semiconductor carrier substrate (102; 202) and said forming of the second array of electrodes (120; 220), removing the top carrier substrate (420; 520) to expose a front surface (440) of the electrode arrangement (100; 200) at which the first array of electrodes (110; 210) is arranged.

13. The method according to claim 12, further comprising bonding the front surface (440) to a temporary wafer (450).

14. The method according to claim 13, further comprising, after said bonding of the front surface (440) to the temporary wafer (450), removing the bottom carrier substrate (402) to expose a back surface (442) of the electrode arrangement (100) at which the second array of electrodes (120) is arranged.

15. The method according to any one of claims 6-14, wherein forming of the first array of electrodes (110; 210) and forming of the second array of electrodes (120; 220) comprises plasma etching.

## Patentansprüche

1. Elektrodenanordnung (100; 200), umfassend:
ein Halbleiterträgersubstrat (102; 202), das eine erste Seitenfläche (104; 204) und eine zweite Seitenfläche (106; 206) gegenüber der ersten Seitenfläche (104; 204) aufweist;
eine erste Matrix aus Elektroden (110; 210), die über der ersten Seitenfläche (104; 204) angeordnet ist;
eine zweite Matrix aus Elektroden (120; 220), die unter der zweiten Seitenfläche (106; 206) angeordnet ist;
elektronische Schaltungen (130; 230) zum Verarbeiten von elektrischen Signalen, die von der ersten Matrix der Elektroden (110; 210) und der zweiten Matrix aus Elektroden (120; 220) aufgezeichnet werden, wobei die elektronischen Schaltungen (130; 230) mindestens eine Schicht umfassen, die über der ersten Seitenfläche (104; 204) und unter der ersten Matrix aus Elektroden (110; 210) angeordnet ist;
eine Verbindungsschicht (140; 240), die über der mindestens einen Schicht der elektronischen Schaltungen (130; 230) angeordnet ist, wobei die Verbindungsschicht (140; 240) dazu konfiguriert ist, die Elektroden (110; 210) der ersten Matrix und die Elektroden (120; 220) der zweiten Matrix mit den elektronischen Schaltungen (130; 230) zu verbinden, wobei die Verbindungsschicht (140; 240) dazu konfiguriert ist, eine erste Verbindung (142a; 242a) in einer Ebene der Verbindungsschicht (140; 240) zwischen einem ersten Punkt (144a; 244a) und einem zweiten Punkt (144b; 244b) bereitzustellen, um eine Elektrode (120; 220) in der zweiten Matrix mit den elektronischen Schaltungen (130; 230) zu verbinden;
eine erste Zusammenschaltung (146; 246) zum elektrischen Verbinden des ersten Punktes (144a; 244a) mit den elektronischen Schaltungen (130; 230);
eine Vielzahl von Substratdurchkontaktierungen, TSVs, (150; 250), die sich durch das Halbleiterträgersubstrat (102; 202) hindurch zwischen der ersten Seitenfläche (104; 204) und der zweiten Seitenfläche (106; 206) erstrecken, um eine elektrische Verbindung durch das Halbleiterträgersubstrat (102; 202) hindurch zu bilden;
eine zweite Zusammenschaltung (148; 248), die sich durch eine Ebene hindurch erstreckt, die von der mindestens einen Schicht der elektronischen Schaltungen (130; 230) definiert ist, wobei die zweite Zusammenschaltung (148; 248) und eine erste TSV (150; 250) der Vielzahl von TSVs den zweiten Punkt (144b; 244b) mit der Elektrode (120; 220) in der zweiten Matrix. elektrisch verbinden.

2. Elektrodenanordnung nach Anspruch 1, wobei die Verbindungsschicht (140) dazu konfiguriert ist, eine zweite Verbindung (142b) in einer Ebene der Verbindungsschicht (140) zwischen einem dritten Punkt (144c) und einem vierten Punkt (144d) zum Verbinden einer Elektrode (110) in der ersten Matrix mit den elektronischen Schaltungen (130) bereitzustellen, wobei die Elektrodenanordnung (100) ferner eine dritte Zusammenschaltung (158) zum elektrischen Verbinden des dritten Punktes (144c) mit den elektronischen Schaltungen (130), eine vierte Zusammenschaltung (160) zum Verbinden des vierten Punktes (144d) mit einer zweiten TSV (154) der Vielzahl von TSVs, eine fünfte Zusammenschaltung (162) zum Verbinden der Elektrode (110) in der ersten Matrix mit einer dritten TSV (156) der Vielzahl von TSVs und eine Überbrückungsstruktur (164), die an der zweiten Seitenfläche (106) zum Verbinden der zweiten TSV (154) mit der dritten TSV (156) angeordnet ist, umfasst.

3. Elektrodenanordnung nach Anspruch 1, wobei die Verbindungsschicht (240) dazu konfiguriert ist, eine zweite Verbindung (242b) in einer Ebene der Verbindungsschicht (240) zwischen einem dritten Punkt (244c) und einem vierten Punkt (244d) zum Verbinden einer Elektrode (220) in der ersten Matrix mit den elektronischen Schaltungen (230) bereitzustellen, wobei die Elektrodenanordnung (200) ferner eine dritte Zusammenschaltung (254) zum elektrischen Verbinden des dritten Punktes (244c) mit den elektronischen Schaltungen (230) und eine vierte Zusammenschaltung (256) zum Verbinden der Elektrode (220) in der ersten Matrix mit dem vierten Punkt (244d) umfasst.

4. Elektrodenanordnung nach einem der vorhergehenden Ansprüche, ferner umfassend eine Back-End-of-Line-Kapazitätsstruktur, die an der zweiten Seitenfläche (106; 206) zwischen der zweiten Matrix aus Elektroden (120; 220) und dem Halbleiterträgersubstrat (102; 202) gebildet ist.

5. Neurale Sonde, wobei die neurale Sonde (300) die Elektrodenanordnung (100; 200) nach einem der vorhergehenden Ansprüche umfasst, wobei das Halbleiterträgersubstrat (102; 202) dazu geeignet ist, in ein Gehirn eingesetzt zu werden.

6. Verfahren zum Herstellen einer Elektrodenanordnung, wobei das Verfahren umfasst:
Bilden von elektronischen Schaltungen (130; 230) über einer ersten Seitenfläche (104; 204) eines Halbleiterträgersubstrats (102; 202), wobei die elektronischen Schaltungen (130; 230) mindestens eine Schicht umfassen;
Bilden einer Vielzahl von metallgefüllten Strukturen (412), die sich von der ersten Seitenfläche (104; 204) aus in das Halbleiterträgersubstrat (102; 202) hinein erstrecken;
Bilden einer Verbindungsschicht (140; 240) über den elektronischen Schaltungen (130; 230) und Bilden von Zusammenschaltungen zwischen der Verbindungsschicht (140; 240) und den elektronischen Schaltungen (130; 230) und zwischen der Verbindungsschicht (140; 240) und den metallgefüllten Strukturen (412), so dass die Verbindungsschicht (140; 240) dazu konfiguriert ist, eine erste Verbindung (142a; 242a) in einer Ebene der Verbindungsschicht (140; 240) zwischen einem ersten Punkt (144a; 244a), der durch eine erste Zusammenschaltung (146; 246) mit den elektronischen Schaltungen (130; 230) verbunden ist, und einem zweiten Punkt (144b; 244b), der durch eine zweite Zusammenschaltung (148; 248), die sich durch eine Ebene hindurch erstreckt, die durch die mindestens eine Schicht der elektronischen Schaltungen (130; 230) definiert ist, mit einer ersten metallgefüllten Struktur (412a) der Vielzahl von metallgefüllten Strukturen (412) verbunden ist, bereitzustellen;
Bilden einer ersten Matrix aus Elektroden (110; 210) über den elektronischen Schaltungen (130; 230);
Ausdünnen des Halbleiterträgersubstrats (102; 202), um eine zweite Seitenfläche (106; 206) des Halbleiterträgersubstrats (102; 202) gegenüber der ersten Seitenfläche (104; 204) zu definieren und einen Boden der metallgefüllten Strukturen (412) freizulegen, so dass die metallgefüllten Strukturen (412) eine Vielzahl von Substratdurchkontaktierungen, TSVs, (150; 250) bilden, die sich durch das Halbleiterträgersubstrat (102; 202) hindurch erstrecken;
Bilden einer zweiten Matrix aus Elektroden (120; 220) an der zweiten Seitenfläche (106; 206) des Halbleiterträgersubstrats (102; 202), wobei eine Elektrode (110; 210) der zweiten Matrix aus Elektroden (120; 220) durch eine erste TSV (150; 250) der Vielzahl von TSVs elektronisch verbunden ist, und die zweite Zusammenschaltung (148; 248) mit dem zweiten Punkt (144b; 244b) in der Verbindungsschicht (140; 240) elektronisch verbunden ist.

7. Verfahren nach Anspruch 6, wobei das Bilden der zweiten Matrix aus Elektroden (120; 220) umfasst:
Verarbeiten eines unteren Trägersubstrats (402), wobei das Verarbeiten das Bilden der zweiten Matrix aus Elektroden (120; 220) auf dem unteren Trägersubstrat (402) und das Definieren von elektrischen Verbindungsstrukturen an einer Oberfläche auf dem unteren Trägersubstrat umfasst, wobei jede elektrische Verbindungsstruktur an einer Elektrode (120; 220) in der zweiten Matrix aus Elektroden (120; 220) angeordnet oder damit elektrisch verbunden ist; und
Bonden der Oberfläche des unteren Trägersubstrats (402) mit einer zweiten Seitenfläche (106; 206) des Halbleiterträgersubstrats (102; 202) durch Hybridbonden.

8. Verfahren nach Anspruch 7, wobei das Bilden der Verbindungsschicht (140) und das Bilden von Zusammenschaltungen das Bilden der Verbindungsschicht (140) umfasst, um eine zweite Verbindung (142b) in einer Ebene der Verbindungsschicht (140) zwischen einem dritten Punkt (144c), der durch eine dritte Zusammenschaltung (158) mit den elektronischen Schaltungen (130) verbunden ist, und einem vierten Punkt (144d), der durch eine vierte Zusammenschaltung (160) mit einer zweiten metallgefüllten Struktur (412b) der Vielzahl von metallgefüllten Strukturen verbunden ist, bereitzustellen, wobei das Verfahren ferner das Bilden einer fünften Zusammenschaltung (162) zum Verbinden einer Elektrode (110) in der ersten Matrix aus Elektroden (110) mit einer dritten metallgefüllten Struktur (412c) der Vielzahl von metallgefüllten Strukturen umfasst.

9. Verfahren nach Anspruch 8, wobei das Verarbeiten auf dem unteren Trägersubstrat (402) das Definieren einer elektrischen Überbrückungsverbindungsstruktur (164) an einer Oberfläche auf dem unteren Trägersubstrat (402) umfasst, wobei die elektrische Überbrückungsverbindungsstruktur (164), wenn die Oberfläche auf dem unteren Trägersubstrat (402) mit der zweiten Seitenfläche (106) des Halbleiterträgersubstrats (102) gebondet wird, eine elektrische Verbindung zwischen einer zweiten TSV (154) und einer dritten TSV (156) bildet, wobei die zweite und die dritte TSV (154, 156) jeweils durch die zweite und die dritte metallgefüllte Struktur (412b, 412c) gebildet werden.

10. Verfahren nach Anspruch 7, wobei das Bilden der ersten Matrix aus Elektroden (210) umfasst:
Verarbeiten eines oberen Trägersubstrats (520), wobei das Verarbeiten das Bilden der ersten Matrix aus Elektroden (210) auf dem oberen Trägersubstrat (520) umfasst, wobei jede Elektrode (210) in der ersten Matrix aus Elektroden (210) an einer elektrischen Verbindungsstruktur an einer Oberfläche auf dem oberen Trägersubstrat (520) angeordnet oder elektrisch damit verbunden ist; und
Bonden der Oberfläche auf dem oberen Trägersubstrat (520) mit einer oberen Oberfläche über der ersten Seitenfläche (206) des Halbleiterträgersubstrats (202) durch Hybridbonden.

11. Verfahren nach einem der Ansprüche 6 bis 9, ferner umfassend das Bilden einer planaren Oxidfläche auf der ersten Matrix aus Elektroden (110; 210) auf dem Halbleiterträgersubstrat (102; 202) und das Bonden einer Oxidfläche (424) auf einem oberen Trägersubstrat (420) mit der planaren Oxidfläche des Halbleiterträgersubstrats (102; 202) durch dielektrisches Bonden.

12. Verfahren nach Anspruch 10 oder 11, ferner umfassend, nach dem Ausdünnen des Halbleiterträgersubstrats (102; 202) und dem Bilden der zweiten Matrix aus Elektroden (120; 220), das Entfernen des oberen Trägersubstrats (420; 520), um eine vordere Oberfläche (440) der Elektrodenanordnung (100; 200) freizulegen, an der die erste Matrix aus Elektroden (110; 210) angeordnet ist.

13. Verfahren nach Anspruch 12, ferner umfassend das Bonden der vorderen Oberfläche (440) mit einem temporären Wafer (450).

14. Verfahren nach Anspruch 13, ferner umfassend, nach dem Bonden der vorderen Oberfläche (440) mit dem temporären Wafer (450), das Entfernen des unteren Trägersubstrats (402), um eine hintere Oberfläche (442) der Elektrodenanordnung (100) freizulegen, an der die zweite Matrix aus Elektroden (120) angeordnet ist.

15. Verfahren nach einem der Ansprüche 6 bis 14, wobei das Bilden der ersten Matrix aus Elektroden (110; 210) und das Bilden der zweiten Matrix aus Elektroden (120; 220) Plasmaätzen umfassen.

## Revendications

1. Agencement d'électrodes (100 ; 200), comprenant :
un substrat porteur semi-conducteur (102 ; 202) ayant une première surface latérale (104 ; 204) et une deuxième surface latérale (106 ; 206) opposée à la première surface latérale (104 ; 204) ;
un premier réseau d'électrodes (110 ; 210) disposé au-dessus de la première surface latérale (104 ; 204) ;
un deuxième réseau d'électrodes (120 ; 220) disposé au-dessous la deuxième surface latérale (106 ; 206) ;
un circuit électronique (130 ; 230) pour traiter les signaux électriques enregistrés par le premier réseau d'électrodes (110 ; 210) et le deuxième réseau d'électrodes (120 ; 220), ledit circuit électronique (130 ; 230) comprenant au moins une couche disposée au-dessus de la première surface latérale (104 ; 204) et au-dessous du premier réseau d'électrodes (110 ; 210) ;
une couche de connexion (140 ; 240) disposée au-dessus de la au moins une couche du circuit électronique (130 ; 230), ladite couche de connexion (140 ; 240) étant configurée pour connecter les électrodes (110 ; 210) du premier réseau et le électrodes (120 ; 220) du deuxième réseau au circuit électronique (130 ; 230), dans lequel la couche de connexion (140 ; 240) est configurée pour fournir une première connexion (142a ; 242a) dans un plan de la couche de connexion (140 ; 240) entre un premier point (144a ; 244a) et un deuxième point (144b ; 244b) pour connecter une électrode (120 ; 220) dans le deuxième réseau au circuit électronique (130 ; 230) ;
une première interconnexion (146 ; 246) pour connecter électriquement le premier point (144a ; 244a) au circuit électronique (130 ; 230) ;
une pluralité de vias traversant le substrat, TSV, (150 ; 250) s'étendant à travers le substrat porteur semi-conducteur (102 ; 202) entre la première surface latérale (104 ; 204) et la deuxième surface latérale (106 ; 206) pour former une connexion électrique à travers le substrat porteur semi-conducteur (102 ; 202) ;
une deuxième interconnexion (148 ; 248) s'étendant à travers un plan défini par la au moins une couche du circuit électronique (130 ; 230), dans lequel la deuxième interconnexion (148 ; 248) et un premier TSV (150 ; 250) de la pluralité de TSV connecte électriquement le deuxième point (144b ; 244b) à l'électrode (120 ; 220) dans le deuxième réseau.

2. Agencement d'électrodes selon la revendication 1, dans lequel la couche de connexion (140) est configurée pour fournir une deuxième connexion (142b) dans un plan de la couche de connexion (140) entre un troisième point (144c) et un quatrième point (144d) pour connecter une électrode (110) dans le premier réseau au circuit électronique (130), l'agencement d'électrodes (100) comprenant en outre une troisième interconnexion (158) pour connecter électriquement le troisième point (144c) au circuit électronique (130), une quatrième interconnexion (160) pour connecter le quatrième point (144d) à un deuxième TSV (154) de la pluralité de TSV, une cinquième interconnexion (162) pour connecter l'électrode (110) dans le premier réseau à un troisième TSV (156) de la pluralité de TSV, et une structure de pont (164) disposée au niveau de la deuxième surface latérale (106) pour connecter le deuxième TSV (154) au troisième TSV (156).

3. Agencement d'électrodes selon la revendication 1, dans lequel la couche de connexion (240) est configurée pour fournir une deuxième connexion (242b) dans un plan de la couche de connexion (240) entre un troisième point (244c) et un quatrième point (244d) pour connecter une électrode (220) dans le premier réseau au circuit électronique (230), l'agencement d'électrodes (200) comprenant en outre une troisième interconnexion (254) pour connecter électriquement le troisième point (244c) au circuit électronique (230), et une quatrième interconnexion (256) pour connecter l'électrode (220) dans le premier réseau au quatrième point (244d).

4. Agencement d'électrodes selon une quelconque des revendications précédentes, comprenant en outre une structure de capacité de fin de ligne formée au niveau de la deuxième surface latérale (106 ; 206) entre le deuxième réseau d'électrodes (120 ; 220) et le substrat porteur semi-conducteur (102 ; 202).

5. Sonde neurale, ladite sonde neurale (300) comprenant l'agencement d'électrodes (100 ; 200) selon une quelconque des revendications précédentes, dans lequel le substrat porteur semi-conducteur (102 ; 202) est adapté pour être inséré dans un cerveau.

6. Procédé de fabrication d'un agencement d'électrodes, ledit procédé comprenant de :
former un circuit électronique (130 ; 230) au-dessus d'une première surface latérale (104 ; 204) d'un substrat porteur semi-conducteur (102 ; 202), ledit circuit électronique (130 ; 230) comprenant au moins une couche ;
former une pluralité de structures remplies de métal (412) s'étendant de la première surface latérale (104 ; 204) dans le substrat porteur semi-conducteur (102 ; 202);
former une couche de connexion (140 ; 240) au-dessus du circuit électronique (130 ; 230) et former des interconnexions entre la couche de connexion (140 ; 240) et les circuits électroniques (130 ; 230) et entre la couche de connexion (140 ; 240) et les structures chargées de métal (412) de sorte que la couche de connexion (140 ; 240) soit configurée pour fournir une première connexion (142a ; 242a) dans un plan de la couche de connexion (140 ; 240) entre un premier point (144a ; 244a) connecté par une première interconnexion (146 ; 246) au circuit électronique (130 ; 230) et un deuxième point (144b ; 244b) connecté par une deuxième interconnexion (148 ; 248), s'étendant à travers un plan défini par la au moins une couche du circuit électronique (130 ; 230), jusqu'à une première structure remplie de métal (412a) de la pluralité de structures remplies de métal (412) ;
former un premier réseau d'électrodes (110 ; 210) au-dessus du circuit électronique (130 ; 230) ;
amincir le substrat porteur semi-conducteur (102 ; 202) pour définir une deuxième surface latérale (106 ; 206) du substrat porteur semi-conducteur (102 ; 202) opposée à la première surface latérale (104 204) et pour exposer un fond des structures remplies de métal (412) de sorte que les structures remplies de métal (412) forment une pluralité de vias traversant le substrat, TSV, (150 ; 250) s'étendant à travers le substrat porteur semi-conducteur (102 ; 202) ;
former un deuxième réseau d'électrodes (120 ; 220) au niveau de la deuxième surface latérale (106 ; 206) du substrat porteur semi-conducteur (102 ; 202), dans lequel une électrode (110 ; 210) du deuxième réseau d'électrodes (120 ; 220) est connectée électriquement par un premier TSV (150 ; 250) de la pluralité de TSV et la deuxième interconnexion (148 ; 248) au deuxième point (144b ; 244b) dans la couche de connexion (140 ; 240).

7. Procédé selon la revendication 6, dans lequel ladite formation du deuxième réseau d'électrodes (120 ; 220) comprend :
le traitement d'un substrat porteur inférieur (402), dans lequel ledit traitement comprend la formation du deuxième réseau d'électrodes (120 ; 220) sur le substrat porteur inférieur (402) et la définition des structures de connexion électrique au niveau d'une surface sur le substrat porteur inférieur, chaque structure de connexion électrique étant disposée au niveau de ou étant connectée électriquement à une électrode (120 ; 220) dans le deuxième réseau d'électrodes (120 ; 220) ; et
la liaison de la surface du substrat porteur inférieur (402) à une deuxième surface latérale (106 ; 206) du substrat porteur semi-conducteur (102 ; 202) par liaison hybride.

8. Procédé selon la revendication 7, dans lequel la formation de la couche de connexion (140) et la formation d'interconnexions comprennent la formation de la couche de connexion (140) pour fournir une deuxième connexion (142b) dans un plan de la couche de connexion (140) entre une troisième point (144c) connecté par une troisième interconnexion (158) au circuit électronique (130) et un quatrième point (144d) connecté par une quatrième interconnexion (160) à une deuxième structure remplie de métal (412b) de la pluralité de structures remplies de métal, dans lequel le procédé comprend en outre la formation d'une cinquième interconnexion (162) pour connecter une électrode (110) dans le premier réseau d'électrodes (110) à une troisième structure remplie de métal (412c) de la pluralité de structures remplies de métal.

9. Procédé selon la revendication 8, dans lequel ledit traitement sur le substrat porteur inférieur (402) comprend la définition d'une structure de connexion de pontage électrique (164) au niveau d'une surface sur le substrat porteur inférieur (402), dans lequel ladite structure de connexion de pontage électrique (164) lorsque la surface sur le substrat porteur inférieur (402) est lié à la deuxième surface latérale (106) du substrat porteur semi-conducteur (102) forme une connexion électrique entre un deuxième TSV (154) et un troisième TSV (156), lequel deuxième et les troisièmes TSV (154, 156) sont formés par les deuxième et troisième structures remplies de métal (412b, 412c), respectivement.

10. Procédé selon la revendication 7, dans lequel ladite formation du premier réseau d'électrodes (210) comprend :
le traitement d'un substrat porteur supérieur (520), dans lequel ledit traitement comprend la formation du premier réseau d'électrodes (210) sur le substrat porteur supérieur (520), chaque électrode (210) dans le premier réseau d'électrodes (210) étant disposée au niveau de ou étant électriquement connectée à une structure de connexion électrique au niveau d'une surface sur le substrat porteur supérieur (520) ; et
la liaison de la surface sur le substrat porteur supérieur (520) à une surface supérieure au-dessus de la première surface latérale (206) du substrat porteur semi-conducteur (202) par liaison hybride.

11. Procédé selon une quelconque des revendications 6 à 9, comprenant en outre la formation d'une surface d'oxyde planaire sur le premier réseau d'électrodes (110 ; 210) sur le substrat porteur semi-conducteur (102 ; 202) et la liaison d'une surface d'oxyde (424) sur un substrat porteur supérieur (420) à la surface d'oxyde planaire du substrat porteur semi-conducteur (102 ; 202) par liaison diélectrique.

12. Procédé selon la revendication 10 ou 11, comprenant en outre après ledit amincissement du substrat porteur semi-conducteur (102, 202) et ladite formation du deuxième réseau d'électrodes (120 ; 220), le retrait du substrat porteur supérieur (420 ; 520) pour exposer une surface avant (440) de l'agencement d'électrodes (100 ; 200) au niveau de laquelle le premier réseau d'électrodes (110 ; 210) est disposé.

13. Procédé selon la revendication 12, comprenant en outre la liaison de la surface avant (440) à une plaquette temporaire (450).

14. Procédé selon la revendication 13, comprenant en outre, après ladite liaison de la surface avant (440) à la plaquette temporaire (450), le retrait du substrat porteur inférieur (402) pour exposer une surface arrière (442) de l'agencement d'électrodes. (100) au niveau duquel le deuxième réseau d'électrodes (120) est disposé.

15. Procédé selon une quelconque des revendications 6 à 14, dans lequel la formation du premier réseau d'électrodes (110 ; 210) et la formation du deuxième réseau d'électrodes (120 ; 220) comprennent une gravure au plasma.
